# EUROPÄISCHE PATENTSCHRIFT

(11) **EP 3 863 461 B1**
(45) Veröffentlichungstag und Bekanntmachung des Hinweises auf die Patenterteilung: **07.12.2022**
(21) Anmeldenummer: 19786764.1
(22) Anmeldetag: 09.10.2019
(51) Int. Cl.: A43B 7/20, A61F 5/01

(54) **FUSSBEWEGUNGSBEGRENZUNGSVORRICHTUNG UND SCHUH**
FOOT MOVEMENT LIMITING DEVICE AND SHOE
DISPOSITIF DE LIMITATION DU MOUVEMENT DU PIED ET CHAUSSURE

(30) Priorität: 09.10.2018 DE 102018124932
(43) Veröffentlichungstag der Anmeldung: 18.08.2021
(73) Patentinhaber: Betterguards Technology GmbH, 10625 Berlin (DE)
(72) Erfinder: BICHLER, Vinzenz, 10777 Berlin (DE); STUMPER, Timo, 12101 Berlin (DE); BUSCHINGER, Oscar, 10707 Berlin (DE)
(74) Vertreter: Okoampah, Rene
(86) Internationale Anmeldenummer: PCT/EP2019/077420
(87) Internationale Veröffentlichungsnummer: WO 2020/074614

(56) Entgegenhaltungen:
- WO-A2-2012/169895
- DE-C1- 4 318 588
- US-A- 4 753 229
- US-A1- 2005 198 869
- US-A1- 2012 029 401
- US-A1- 2016 113 802

## Beschreibung

### Technisches Gebiet

Die vorliegende Erfindung betrifft eine Fußbewegungsbegrenzungsvorrichtung zum Begrenzen einer Fußbewegung über das Sprunggelenk und einen entsprechend ausgebildeten Schuh zum Begrenzen einer Fußbewegung über das Sprunggelenk.

### Stand der Technik

Es ist bekannt, die Bewegung des Sprunggelenks mittels Vorrichtungen zum Begrenzen von Fußbewegungen zu stabilisieren, um Traumata infolge eines Umknickens, das heißt einer Bewegung des Sprunggelenks über zumindest eines seiner Sprunggelenksachsen in einem unphysiologischen Bereich entgegenzuwirken. Die häufigste Form des Umknicktraumas ist eine Sprunggelenksdistorsion als Dehnung oder Ruptur infolge einer Inversion. Inversionsbewegungen über eine Sprunggelenksachse resultieren aufgrund des ansteigenden Inversionswinkels in einer Änderung des Abstands zwischen Fuß und Unterschenkel. Bei Überschreiten eines bestimmten Inversionswinkels oder einer bestimmten Inversionsgeschwindigkeit beziehungsweise Inversionsbeschleunigung kann es zu Verletzungen des Bandapparats des Sprunggelenks oder zu Rupturen kommen.

Um dies zu verhindern, sind Vorrichtungen bekannt, die in einem bestimmten Umfang Bewegungen ermöglichen und ab einem bestimmten Grenzwinkel der Bewegung um die Sprunggelenksachse eine Bewegung gänzlich verhindern. Hierzu sind einerseits relativ starre Orthesen bekannt, bei denen das Hemmen von Bewegungen unter Verwendung von Schienen oder Schienenplatten im Vordergrund steht.

Fernerhin sind Vorrichtungen beziehungsweise Schuhe bekannt, bei welchen eine Bewegung des Sprunggelenks bis zu einem bestimmten Grenzwinkel der Bewegung zugelassen wird, und aufgrund des Aufbaus ab diesem Grenzwinkel eine Bewegung gänzlich blockiert wird. Eine entsprechende Vorrichtung ist beispielsweise aus der EP 2 717 809 B1 bekannt.

Ferner sind Vorrichtungen bekannt, welche im angezogenen Zustand stets ein Mindestmaß an Bewegung zulassen, bei gefährlichen Bewegungen jedoch blockieren. Aus der DE 10 2014 107 335 A1 ist eine Vorrichtung zum adaptiven Begrenzen einer Inversionsbeziehungsweise Supinationsbewegung über das Sprunggelenk gezeigt. Die darin gezeigte Sprunggelenksorthese benötigt, um zu verhindern, dass sich der obere Teil der Orthese bei einer Krafteinwirkung von Fuß kommend in Richtung Fuß verschiebt, zur Abstützung der Aufnahme des oberen Bereichs einen auf der medialen Seite des Sprunggelenks gelegenen druckstabilen Balken, welcher sich zwischen dem oberen Bereich der Orthese und dem unteren Bereich der Orthese erstreckt und fest mit beiden Bereichen verbunden sein muss. Der Balken muss dabei derart ausgebildet sein, dass er die über die Auszugsvorrichtung auf der lateralen Seite in den oberen Bereich eingeleiteten Zugkräfte als Druckkräfte auf der medialen Seite wieder in den unteren Bereich zurückleitet, ohne sich zu Verformen und zu knicken. Dadurch ist zwar eine Positionssicherung in distaler Richtung erzielt. Jedoch beschränkt der Balken auf der medialen Seite des Fußes weiterhin die Bewegungsfreiheit des Sprunggelenks.

Weiterhin ist es bekannt, anstelle der vorgenannten Abstützung zwischen oberen und unteren Teil an dem oberen Teil eine Fixierung zum Fixieren des oberen Teils an einem Unterschenkel vorzusehen. Durch die Fixierung stützt sich der obere Teil in diesem Fall von oben auf dem Knöchel ab. Durch die Abstützung und die Fixierung weisen diese Vorrichtungen ein hohes Gewicht sowie einen relativ starren Aufbau und einen geringen Tragekomfort auf.

Es sind zudem neuere Vorrichtungen beziehungsweise Schuhe bekannt, bei welchen eine Stützanordnung vorgesehen ist, welche sich am Unterschenkel abstützt, indem ein umlaufendes Band in einer Öse umgelenkt, festgezogen und anschließend fixiert wird. Diese Stützanordnung ist über ein Dämpfungselement zum Dämpfen einer Relativbewegung mit einer Halteanordnung zum Halten an einem Fuß verbunden. Das Dämpfungselement ist dabei zumindest einseitig stets fest mit dem Schuh beziehungsweise der Vorrichtung verbunden. Ferner ist stets eine Vorspannungsanordnung händisch nach dem Anziehen der Vorrichtung beziehungsweise des Schuhs vorzuspannen, um im angezogenen Zustand an einem Fuß eine Vorspannung des Dämpfungselements sowie eine hinreichende Wirkung des Dämpfungselements, mithin der Vorrichtung beziehungsweise des Schuhs, bereitzustellen.

Die vorstehend genannten Vorrichtungen müssen jedoch, um ihre Funktion vollständig entfalten zu können, auf die Abmessungen des Unterschenkels des Trägers abgestimmt sein. Befinden sich einzelne Bestandteile der Vorrichtung im Tragezustand nicht an der dafür vorgesehenen Position am Körper des Trägers, kann dies die schützende Wirkung der Vorrichtung reduzieren. Im Extremfall einer ungeeigneten Positionierung kann die Schutzfunktion gar vollständig ausfallen. Bislang wird diesem Problem durch das Bereitstellen von Begrenzungsvorrichtungen in unterschiedlichen Größen oder durch individuelle Anpassungen begegnet. Gerade für den Fall, dass die Begrenzungsvorrichtungen in Schuhe integriert werden sollen, stellt dies ein besonderes Problem dar, da es nun zusätzlich zur Schuhgröße auch auf die Abmessungen des Unterschenkels des Trägers ankommt.

Die DE 43 18 588 C1 zeigt eine Sprunggelenkorthese mit U-förmiger Gelenkmanschette und biegsamen Steg, welche die Merkmale des Oberbegriffs von Anspruch 1 enthält.

### Darstellung der Erfindung

Ausgehend von dem bekannten Stand der Technik ist es eine Aufgabe der vorliegenden Erfindung, eine verbesserte Fußbewegungsbegrenzungsvorrichtung zum Begrenzen einer Fußbewegung über das Sprunggelenk bereitzustellen.

Die Aufgabe wird durch eine Fußbewegungsbegrenzungsvorrichtung zum Begrenzen einer Fußbewegung über das Sprunggelenk mit den Merkmalen des Anspruchs 1 gelöst. Vorteilhafte Weiterbildungen ergeben sich aus den Unteransprüchen, der Beschreibung und den Figuren. Entsprechend wird eine Fußbewegungsbegrenzungsvorrichtung zum Begrenzen einer Fußbewegung über das Sprunggelenk vorgeschlagen, umfassend eine Stützanordnung zum Abstützen an einem Unterschenkel oder von proximal am Sprunggelenk-Knöchel, eine Halteanordnung zum Halten an einem Fuß, bevorzugt einem Fußsohlenbereich, und mindestens ein Anbindungselement zum Begrenzen einer Relativbewegung zwischen der Stützanordnung und der Halteanordnung, wobei das mindestens eine Anbindungselement an zwei verschiedenen Anbindungsstellen an die Stützanordnung angebunden ist. Ferner umfasst die Stützanordnung einen sich von einer lateralen Seite von der ersten Anbindungsstelle posterior herum zumindest auf eine mediale Seite zur zweiten Anbindungsstelle erstreckenden, zugsteifen Bereich, oder einen sich von einer lateralen Seite von der ersten Anbindungsstelle anterior herum zumindest auf eine mediale Seite zur zweiten Anbindungsstelle erstreckenden, zugsteifen Bereich, wobei der zugsteife Bereich an seinen Enden durch einen zugelastischen Bereich verbunden ist, wobei sich der zugelastische Bereich zwischen der ersten Anbindungsstelle und der zweiten Anbindungsstelle entgegengesetzt zum zugsteifen Bereich erstreckt.

Erfindungsgemäß ist das mindestens ein Anbindungselement an einem Punkt über eine gelenkige Halterung an der Halteanordnung gehalten, wobei die gelenkige Halterung derart ausgebildet ist, dass das Anbindungselement in einem vorgegebenen Bereich verschiebbar zur Halteanordnung an diese angebunden ist

Dadurch, dass der zugsteife Bereich an seinen Enden durch einen zugelastischen Bereich verbunden ist, kann der Stützbereich am Körper der die Fußbewegungsbegrenzungsvorrichtung tragenden Person durch den zugelastischen Bereich in Position gehalten werden. Dadurch kann sichergestellt werden, dass die Fußbewegungsbegrenzungsvorrichtung von Anwendern mit unterschiedlichen Beinumfängen, insbesondere unterschiedlichen Fesselumfängen Knöchelumfängen, im Bereich oberhalb des Knöchels getragen werden kann. Die Komponenten der Fußbewegungsbegrenzungsvorrichtung können aufgrund des flexiblen Bereichs stets derart in Position gehalten werden, dass das Potential der Fußbewegungsbegrenzungsvorrichtung voll ausgeschöpft werden kann. Einer unsachgerechten Positionierung, die auf die Körpermaße des Anwenders, insbesondere auf dessen Fesselumfang und Knöchelumfang, zurückzuführen ist, kann somit entgegengewirkt werden.

Darüber hinaus kann der zugelastische Bereich zumindest teilweise eine Vorspannung bereitstellen, welche wiederum auf die Steifigkeit der Stützanordnung einen erhöhenden Einfluss haben kann.

Der zugelastische Bereich weist hierfür vorzugsweise im unbelasteten Zustand eine Länge auf, welche in Verbindung mit der Länge des zugsteifen Bereichs in Umfangsrichtung des Unterschenkels gesehen einen Umfang der Stützanordnung ausbildet, welcher kleiner ist als ein vorgegebener minimaler Fesselumfang, für welchen die Vorrichtung ausgelegt ist. Dadurch erfährt der zugelastische Bereich im angelegten bzw. angezogenen Zustand der Fußbewegungsbegrenzungsvorrichtungen einem Fuß eine Dehnung, welche die Vorspannung des zugelastischen Bereichs bewirkt.

Alternativ kann der zugelastische Bereich an einem Ende auch mittels einer Öse und einer Schlaufe an das entsprechende Ende des zugsteifen Bereichs angebracht werden, wobei bevorzugt durch ein Anpassen der Schlaufenlänge eine Vorspannung des zugelastischen Bereichs einstellbar ist. Ferner kann der zugsteife Bereich an zumindest einem Ende eine Reihe von in Umfangsrichtung beabstandet voneinander angeordneter Ösen aufweisen, wobei ein an Ende des zugelastischen Bereichs befestigter Haken in eine der Ösen eingehakt werden kann. Alternativ kann auch der zugelastische Bereich eine Öse und der zugsteife Bereich an dessen Ende eine Reihe von Haken aufweisen.

Ferner kann durch das Vorsehen des zugelastischen Bereichs eine Anpassung an verschiedene Knöchelumfänge und Fesselumfänge erleichtert werden. Durch den zugelastischen Bereich kann sich der Stützbereich zumindest zwischen einem vorgegebenen minimalen theoretischen Fesselumfang und einem maximalen theoretischen Fesselumfang an die Fessel des die Vorrichtung tragende Person anpassen. Mithin ist die Fußbewegungsbegrenzungsvorrichtung für Personen mit verschiedenen Fesselumfänge geeignet, ohne dass die Vorrichtung einer Anpassung bedarf. Ferner kann sich die Stützvorrichtung trotz der Tatsache, dass jede Person eine anders ausgebildete untere Extremität hat, die Vorrichtung sich im Wesentlichen passgenau an den Fesselbereich der Person anlegen.

Zudem kann durch den sich zwischen den Enden des zugsteifen Bereichs erstreckenden zugelastischen Bereich ein Einstieg in die Fußbewegungsbegrenzungsvorrichtung ermöglicht werden, insbesondere wenn die Fußbewegungsbegrenzungsvorrichtung Bestandteil eines Schuhes, einer Bandage oder eines Sockens ist oder als Orthese ausgebildet ist.

Dadurch dass die Stützanordnung einen sich von einer lateralen Seite von der ersten Anbindungsstelle posterior herum zumindest auf eine mediale Seite zur zweiten Anbindungsstelle erstreckenden, zugsteifen Bereich umfasst, ist die Fußbewegungsbegrenzungsvorrichtung dazu geeignet, mindestens ein Außendband des Anwenders, insbesondere das Ligamentum tibiofibulare anterius und/oder des Ligamentum fibulocalcaneare, zu schützen, sofern die gelenkige Halterung an einer lateralen Seite des Sprunggelenks angeordnet ist.

Alternativ oder zusätzlich kann die Fußbewegungsbegrenzungsvorrichtung gemäß einer weiter bevorzugten Ausführungsform derart ausgebildet sein, dass eine Stützanordnung einen sich von einer medialen Seite von der ersten Anbindungsstelle posterior herum zumindest auf eine laterale Seite, und bevorzugt anterior herum wieder auf die mediale Seite, zur zweiten Anbindungsstelle erstreckenden, zugsteifen Bereich umfasst. Dies kann insbesondere vorteilhaft sein, wenn die gelenkige Halterung an einer medialen Seite der Fußbewegungsbegrenzungsvorrichtung insbesondere zum Schutz eines Innenbands des Sprunggelenks angeordnet ist.

Dadurch, dass das mindesten eine Anbindungselement an einem Ende gelenkig an der Halteanordnung gehalten ist und das Anbindungselement an die Stützanordnung, bevorzugt beweglich, angebunden ist, ist ermöglicht, dass sich das Anbindungselement bei einer Bewegung des Fußes relativ zum Unterschenkel über das Sprunggelenk relativ zum Fuß bewegen kann. Mit anderen Worten kann sich das Anbindungselement um die gelenkige Halterung bewegen, sodass die Ausrichtung des Anbindungselements bezogen auf Unterschenkel und Vorfuß, wenn die Fußbewegungsbegrenzungsvorrichtung an einem Fuß angezogen ist, ihre relative Position verändern kann. Dadurch ist zum einen die Bewegungsfreiheit des Fußes deutlich vergrößert, da ein zumindest einseitig fest an der Vorrichtung befestigtes Anbindungselement eine Strukturversteifung der Vorrichtung darstellt, was durch die gelenkige Halterung vermieden ist.

Zudem kann das Anbindungselement dadurch, dass es sich bei einer Bewegung des Fußes über das Sprunggelenk stets neu ausrichten kann, näher an eine optimale Kraftübertragungsrichtung, welche sich bei einer Inversion aufgrund der Rückhaltekraft zwischen Stützanordnung und Halteanordnung einstellt, ausrichten. Mithin ist einerseits erzielt, dass das Anbindungselement eine besonders effiziente Rückhaltung bereitstellen kann.

Mithin kann eine derart ausgebildete Fußbewegungsbegrenzungsvorrichtung eine im Vergleich zu herkömmlichen Vorrichtungen verbesserte Schutzwirkung und gleichzeitig eine verbesserte Bewegungsfreiheit und einen verbesserten Tragekomfort bereitstellen.

Es hat sich herausgestellt, dass ein gesondertes Festziehen und Fixieren der Stützanordnung am Unterschenkel nicht erforderlich ist, und eine Schutzwirkung für das Sprunggelenk auch ohne eine derartige Maßnahme gegeben ist.4

Der Begriff Fußbewegungsbegrenzungsvorrichtung meint eine Vorrichtung, welche im an einen Fuß angelegten bzw. angezogenen Zustand zumindest eine Bewegung in zumindest einer (zusammengesetzten) Bewegungsrichtung bis zu einem vorgegebenen Wert bzw. vorgegebenen Umfang der Bewegung zugelassen wird, bevorzugt ohne signifikante Einschränkung der Bewegungsfreiheit. Oberhalb des vorgegebenen Wertes bzw. vorgegebenen Umfangs der Bewegung erfolgt eine Restriktion der Bewegung durch die Vorrichtung. Der Bewegungsumfang in dieser zumindest einen Bewegungsrichtung ist mithin durch die Vorrichtung begrenzt.

Die Begriffe lateral, medial, posterior, anterior, proximal, distal, dorsal und plantar sind derart zu verstehen, dass sie den anatomischen Richtungsbezeichnungen entsprechen, wenn die Fußbewegungsbegrenzungsvorrichtung beziehungsweise der Schuh korrekt an einen Fuß angezogen ist.

Der Begriff "laterale Seite" umfasst folglich vorliegend eine Außenseite der Fußbewegungsbegrenzungsvorrichtung. Hierbei entspricht die laterale Seite der Fußbewegungsbegrenzungsvorrichtung einer lateralen Seite eines menschlichen Fußes beziehungsweise einer unteren menschlichen Extremität, wenn dieser den Schuh trägt. Die "laterale Seite" ist in im getragenen Zustand der Fußbewegungsbegrenzungsvorrichtung seitlich beziehungsweise von der Körpermitte des Trägers abgewandt. Mit anderen Worten umfasst der Begriff "laterale Seite" eine laterale Seite des menschlichen Körpers im Sinne der (topographischen) Anatomie.

Entsprechend umfasst der Begriff "mediale Seite" vorliegend eine Innenseite der Fußbewegungsbegrenzungsvorrichtung. Die mediale Seite der

Fußbewegungsbegrenzungsvorrichtung entspricht einer medialen Seite eines menschlichen Fußes beziehungsweise einer unteren menschlichen Extremität, wenn dieser die Fußbewegungsbegrenzungsvorrichtung trägt. Die "mediale Seite" ist in im getragenen Zustand der Fußbewegungsbegrenzungsvorrichtung zur Körpermitte hin orientiert beziehungsweise in der Mitte gelegen. Mit anderen Worten umfasst der Begriff "mediale Seite" eine mediale Seite des menschlichen Körpers im Sinne der (topographischen) Anatomie.

Ferner entspricht der Begriff "dorsal" einer Oberseite des Fußes, der Begriff "plantar" einer Unterseite des Fußes, der Begriff "proximal" zum Körperzentrum hin weisend beziehungsweise gelegen, der Begriff "distal" vom Körperzentrum entfernt weisend beziehungsweise gelegen.

Ein "Fersenbereich" umfasst einen Bereich der Fußbewegungsbegrenzungsvorrichtung beziehungsweise eines Schuhs, in welchem im getragenen Zustand eine Ferse eines Fußes aufgenommen wird. Mithin entspricht der Fersenbereich einer hinteren beziehungsweise posterioren Seite der Fußbewegungsbegrenzungsvorrichtung im Sinne der (topographischen) Anatomie des menschlichen Körpers. Folglich liegt der Schuhfersenbereich einer anterioren Seite gegenüber.

Unter "zugsteif" ist hier jedes Material zu verstehen, mit welchem Zugkräfte übertragen werden können. Zugsteife Materialien in Sinne der Anmeldung können ein gewisses Anfangsdehnungsvermögen aufweisen und bevorzugt ab einer bestimmten Dehngrenzwert oder Streckgrenzwert derart versteifen, dass das Dehnungsvermögen dann im Vergleich zum Anfangsdehnungsvermögen beziehungsweise Streckungsvermögen stark reduziert ist. Als zugsteifes Band kann vorliegend etwa ein Textilgeflecht aus Fäden eines zugsteifen Materials Anwendung finden, wobei bei einer Zugbelastung auf das Textilgeflecht anfangs die Fäden des Textilgeflechts eine zunehmende Ausrichtung in der Längserstreckung des Textilgeflechts erfahren, so dass Anfangs durch die Ausrichtung der Fäden ein hohes Anfangsdehnungsvermögen bereitgestellt ist, und nach dem Ausrichten der Fäden im Wesentlichen in Kraftflussrichtung das Band eine zugsteife Struktur aufweist. Alternativ kann das Material auch im Wesentlichen unmittelbar zugsteifes Verhalten aufweisen.

Der Begriff "Sprunggelenk" umfasst vorliegend das obere und das untere Sprunggelenk und entsprechend die Bewegungsachse des oberen Sprunggelenks, welche im Wesentlichen die Plantarflexion und Dorsalextension des Fußes ermöglicht, und die Bewegungsachse des unteren Sprunggelenks, welche im Wesentlichen die Inversion und Eversion, sowie Supination, Adduktion und Plantarflexion, Abduktion und Dorsalextension, ermöglicht.

Unter dem Begriff "Knöchel" wird hier ferner die Ausprägung der Gelenkpfanne, der Malleolengabel des oberen Sprunggelenks verstanden. Folglich umfasst der Begriff "Knöchel" vorliegend den Außenknöchel, welcher durch die Ausprägung des Malleolus lateralis ausgebildet ist, und den Innenknöchel, welcher durch die Ausprägung des Malleolus medialis ausgebildet ist. Aufgrund der Ausbildung der Malleolengabel als Gelenkpfanne weist der Knöchel im Vergleich zum sich oberhalb, folglich proximal anschließenden Abschnitt des Unterschenkels in Bezug auf die proximal-distale Richtung eine größere Querschnittsfläche auf. Mithin ist der Umfang des Knöchels im Vergleich zum sich proximal anschließenden Abschnitt größer.

Unter einer gelenkigen Halterung beziehungsweise "gelenkig gehalten" wird hier insbesondere verstanden, dass das Anbindungselement an einer Verbindungsstelle zur Halteanordnung zumindest einen Freiheitsgrad aufweist. Bevorzugt ist das Anbindungselement dabei um eine Schwenkachse herum, bevorzugt zumindest in einem vorgegebenen Winkelbereich, schwenkbar.

Unter dem Begriff "Band" wird insbesondere ein längliches Element verstanden, dass einerseits zugsteif ist, mithin Zugkräfte übertragen kann, und andererseits quer zur Längserstreckung des Bandes biegeweich beziehungsweise flexibel ist, so dass es quer zu seiner Längserstreckung an eine entsprechende Struktur anlegbar ist. Zudem kann ein Band in einfacher Weise umgelenkt werden. Der Begriff Band umfasst vorliegend allgemein ein längliches, biegeschlaffes, optional elastisches Element, welches die Form einer einzelnen Faser, eines Faserstrangs, eines Drahts, einer Kordel, eines Seils, eines Textilgewebes mit begrenzter Breite und festen, beidseitigen Webkanten oder dergleichen aufweisen kann.

Gemäß einer weiter bevorzugten Ausführungsform ist das mindestens eine Anbindungselement an zwei verschiedenen Anbindungsstellen an der Stützanordnung, bevorzugt beweglich, besonders bevorzugt verschiebbar, angebunden. Das Anbindungselement kann sich dadurch stets entlang des Anbindungselements innerhalb der beiden Anbindungsstellen verschieben und so bei Bewegungen des Fußes über das Sprunggelenk stets die oben beschriebene günstige beziehungsweise gar optimale Ausrichtung einnehmen.

Es hat sich dabei herausgestellt, dass wenn sich das zumindest eine Anbindungselement bevorzugt von einer Anbindungsstelle zur anderen Anbindungsstelle erstreckt und das Anbindungselement zwischen den beiden Anbindungsstellen verschiebbar an der Halteanordnung gehalten ist, die oben genannte Wirkung in besonders ausgeprägter Weise erhalten lässt.

Bevorzugt ist das Anbindungselement als längliches, zugsteifes Element, bevorzugt in Form eines Bandes, einer Schnur, eines Fadens, eines Seils, eines Drahtes, und/oder eines Gewirkes, bevorzugt eines Textilgewirkes, bereitgestellt.

Als weiterhin vorteilhaft hat sich eine Ausführung herausgestellt, bei der die Anbindungsstellen relativ zum Anbindungselement derart positioniert sind, dass eine infolge einer Fußbewegung über das Sprunggelenk im Anbindungselement entstehende Rückhaltekraft in eine lateral-proximal wirkende Komponente und eine medial-proximal wirkende Komponente aufgeteilt wird. Dadurch ist es insbesondere möglich, die durch das Anbindungselement erzeugte Rückhaltekraft beidseits des Fußes beziehungsweise Unterschenkels einer die Fußbewegungsbegrenzungsvorrichtung tragenden Person in die Stützanordnung eingeleitet werden können, sodass sich gegenüberliegend des Anbindungselements eine Abstützung der Stützanordnung am Unterschenkel beziehungsweise Knöchel in Form einer Linienlast ergibt. Mit anderen Worten erfolgt die Abstützung dadurch, dass an der Stützanordnung beidseitig angezogen wird und sich die Stützanordnung dadurch im Wesentlichen ohne verschoben zu werden an dem Knöchel abstützen kann.

In einer weiter bevorzugten Ausführungsform ist das mindestens eine Anbindungselement über ein Dämpfungselement an der Stützanordnung oder der Halteanordnung gehalten.

Das Dämpfungselement kann im Vergleich zu herkömmlichen Vorrichtungen nochmals kleiner dimensioniert werden, da die durch das Dämpfungselement bereitgestellte Dämpfungswirkung beziehungsweise Dämpfungskraft optimal genutzt werden kann.

Es hat sich gezeigt, dass aufgrund der oben genannten verbesserten Ausrichtung des Dämpfungselements und der verbesserten Schutzwirkung des Dämpfungselements sogar auf eine händische Vorspannung des Dämpfungselements verzichtet werden kann.

Unter einem "Dämpfungselement" wird eine Vorrichtung verstanden, welche geschwindigkeitsabhängig, beziehungsweise beschleunigungsabhängig eine Dämpfung einer Relativbewegung zwischen zwei Komponenten des Dämpfungselements bereitstellt. Mithin ist durch das Dämpfungselement eine Relativbewegung zwischen der Stützanordnung und der Halteanordnung geschwindigkeitsabhängig beziehungsweise beschleunigungsabhängig dämpft.

Bevorzugt ist das Dämpfungselement dabei als adaptiv dämpfendes Dämpfungselement bereitgestellt. Mit anderen Worten weist das Dämpfungselement unterhalb einer vorgegebenen Grenzgeschwindigkeit beziehungsweise Grenzbeschleunigung eine bevorzugt niedrige erste Dämpfungskonstante auf und ab beziehungsweise jenseits der vorgegebenen Grenzgeschwindigkeit beziehungsweise Grenzbeschleunigung eine bevorzugt höhere Dämpfungskonstante auf. Dadurch ist es möglich, dass das Dämpfungselement unterhalb der vorgegebenen Grenzgeschwindigkeit beziehungsweise Grenzbeschleunigung eine geringere Dämpfungswirkung entsprechend des Verhältnisses der Dämpfungskonstanten bereitstellt, als oberhalb der Grenzgeschwindigkeit beziehungsweise Grenzbeschleunigung. Es hat sich gezeigt, dass hierdurch insbesondere bei Bewegungen mit moderaten Geschwindigkeiten beziehungsweise Beschleunigungen so eine nahezu ungehinderte Beweglichkeit des Sprunggelenks bereitgestellt werden kann. Tritt eine Inversion mit hohen Geschwindigkeiten und/oder Beschleunigungen auf, bei welchen die Gefahr von Verletzungen des Bandapparats des Sprunggelenks besteht, dämpft die Fußbewegungsbegrenzungsvorrichtung über das das Dämpfungselement die Inversionsbewegung und eine Verletzung wird vermieden.

In einer bevorzugten Weiterbildung ist das Dämpfungselement an dem zugsteifen Bereich der Stützanordnung befestigt.

In einer weiter bevorzugten Ausführungsform ist das Dämpfungselement am zumindest einen Anbindungselement verschiebbar gehalten, wobei das Dämpfungselement zwischen den beiden Anbindungsstellen an das Anbindungselement angebunden ist.

Gemäß einer weiter bevorzugten Ausführungsform sind eine Mehrzahl von Anbindungselementen, bevorzugt zwei Anbindungselemente vorgesehen. Dadurch ist insbesondere eine besonders günstige Geometrie bezüglich einer Kraftübertragung beziehungsweise Kraftweiterleitung von dem Dämpfungselement in die Anbindungselemente und weiter in die Stützanordnung erzielt. Die durch das Dämpfungselement erzeugte Dämpfungskraft, welche im Wesentlichen in Richtung des Mittelsplines wirkt, kann so je nach Orientierung anteilig an die bevorzugt beiden Anbindungselemente übertragen werden. Bevorzugt erstreckt sich jedes der Anbindungselemente von einer Anbindungsstelle zu einer anderen Anbindungsstelle, wobei das Dämpfungselement jeweils zwischen den beiden Anbindungsstellen an das jeweilige Anbindungselement bevorzugt verschiebbar angebunden ist. Die einzelnen Anbindungselemente können dabei jeweils eigene Anbindungsstellen aufweisen, oder sich an gemeinsamen Anbindungsstellen treffen.

Dabei ist bevorzugt ein erstes Anbindungselement bezogen auf einen Mittelspline, bevorzugt eine Mittelachse, des Dämpfungselements beabstandet von dem Mittelspline, bevorzugt der Mittelachse, auf einer ersten Seite bevorzugt gleitend an das Dämpfungselement angebunden, und das zweite Anbindungselement bezogen auf den Mittelspline, bevorzugt die Mittelachse, des Dämpfungselements beabstandet von dem Mittelspline, bevorzugt der Mittelachse, auf einer zweiten Seite bevorzugt gleitend an das Dämpfungselement angebunden. Dieser Aufbau führt zu einer nochmals verbesserten Ausrichtbarkeit des Dämpfungselements, um sich so nahe wie möglich beziehungsweise so direkt wie möglich in Kraftrichtung zu orientieren.

Wenn der Abstand der Anbindungen von erstem Anbindungselement und zweitem Anbindungselement zum Dämpfungselement bezogen zum Mittelspline, bevorzugt zur Mittelachse, im Wesentlichen gleich groß ist, sind entsprechend auch die über den Abstand der Anbindungen in Bezug auf den Mittelspline gegensätzlich wirkenden Momente ähnlich hoch. Mithin ist hierdurch ein besonders effizientes Orientieren des Dämpfungselements in Richtung einer direkten Kraftflussrichtung ermöglicht.

Um den Aufbau und ferner der Fußbewegungsbegrenzungsvorrichtung weiter zu vereinfachen, können gemäß einer weiter bevorzugten Ausführungsform das erste Anbindungselement und das zweite Anbindungselement als Einzelteil ausgebildet sein, wobei das Einzelteil dann an der ersten Anbindungsstelle oder der Anbindungsstelle umgelenkt und gleitend geführt ist.

Es hat sich herausgestellt, dass wenn eine Tasche zum zumindest teilweisen Aufnehmen des Anbindungselements und/oder des Dämpfungselements vorgesehen ist, wobei das in der Tasche aufgenommene Anbindungselement und/oder Dämpfungselement in der Tasche relativ zu dieser beweglich angeordnet ist, sich die Beweglichkeit des Dämpfungselements relativ zumindest zur Halteanordnung erleichtert ist. Mit anderen Worten kann durch die Tasche zumindest teilweise ein Raum für die freie Bewegung des Dämpfungselements bereitgestellt werden. Zudem kann die Tasche als Schutz für zumindest Teile des Dämpfungselements fungieren. Als besonders vorteilhaft hat sich dabei eine sich proximal erweiternde Dreieck-förmige beziehungsweise Trapez-förmige Form der Tasche gezeigt.

Um die freie Beweglichkeit des Dämpfungselements in der Tasche zu erzielen, kann die Tasche bevorzug ein Material mit einem niedrigen Reibungskoeffizienten aufweisen, wobei die Tasche bevorzugt mit der Halteanordnung verbunden ist. Vorzugsweise weist die Tasche ein Aramid, bevorzugt PPTA, oder ein Polyolefin, bevorzugt PTFE, auf, oder das Material der Tasche weist zumindest teilweise eine reibungsarme Beschichtung auf. Reibungsarme Beschichtungen sind an sich bekannt.

Gemäß einer weiter bevorzugten Ausführungsform ist eine Umhüllung vorgesehen, welche das Dämpfungselement und bevorzugt das zumindest eine Anbindungselement zumindest teilweise umhüllt, wobei die Umhüllung bevorzugt zumindest teilweise innerhalb der Tasche relativ zur Tasche beweglich angeordnet ist. Hierdurch kann unter anderem der Schutz des Dämpfungselements beziehungsweise von sensiblen Komponenten des Dämpfungselements weiter gesteigert werden. Zudem kann die Umhüllung bevorzugt eine Positionierung des Dämpfungselements und des zumindest einen Anbindungselements relativ zueinander bereitzustellen.

Gemäß einer weiter bevorzugten Ausführungsform kann die gelenkige Halterung als Drehgelenk und/oder Kugelgelenk ausgebildet sein, welches an einer festen Stelle der Halteanordnung angeordnet ist. Dadurch kann erzielt werden, dass die Krafteinleitung von dem Anbindungselement in die Halteanordnung stets in einem Zustand, in welchem die Vorrichtung an einen Fuß angebracht ist, in einer in Bezug zum Fuß vorgegebenen Position in die Halteanordnung und über diese in den Fuß übertragen wird. Die Position ist dabei bevorzugt so gewählt, dass die Kraftrichtung einer durch die Vorrichtung bereitgestellten Rückhaltekraft zu einer Inversionsbewegung oder einer Supinationsbewegung ausgerichtet ist.

Alternativ oder zusätzlich kann die gelenkige Halterung derart ausgebildet sein, dass das Anbindungselement in einem vorgegebenen Bereich verschiebbar zur Halteanordnung an diese angebunden ist.

Als besonders vorteilhaft hat sich eine optionale Ausführungsform der gelenkigen Halterung herausgestellt, bei welcher die Halteanordnung ein Band aufweist, dass an zwei voneinander beabstandeten Stellen an der Halteanordnung befestigt ist und dadurch eine Schlaufe oder Öse ausbildet. Hierbei ist das Anbindungselement, bevorzugt über eine Öse oder Schlaufe, durch welche das Band verläuft, an dem Band gehalten. Alternativ kann das Anbindungselement auch direkt in dem Band geführt sein, oder die Halteanordnung weist eine Öse auf, durch welche das Anbindungselement verläuft und somit geführt ist. Dadurch kann sich das Anbindungselement zum einen zumindest teilweise entlang des Bandes bzw. der Öse relativ zur Halteanordnung verschieben. Ferner ist es bei dieser bevorzugten Ausführungsform möglich, dass das Anbindungselementrelativ zur Halteanordnung verschwenkt werden kann.

Bevorzugt kann über die Schlaufenausbildung eine Öffnung der Stützanordnung eingestellt werden. In einem losen Zustand der Schlaufe kann so ein Anlegen der Stützanordnung in einfacher Weise erfolgen. Nachdem die Stützanordnung über den Knöchel in seine Position posterior zu diesem gebracht worden ist, kann durch Anziehen der Schlaufe über die Öse die Stützanordnung eng an die untere Extremität des die Fußbewegungsbegrenzungsvorrichtung tragenden Person angelegt und fixiert werden.

Gemäß einer weiter bevorzugten Ausführungsform weist das Dämpfungselement eine mit einem Dämpfungsmedium, bevorzugt einem Dämpfungsfluid, gefüllte Aufnahme auf, in der ein Auszugskörper relativ zu dieser beweglich aufgenommen ist, wobei der Auszugskörper mit einer sich in einer Auszugsrichtung aus der Aufnahme erstreckenden Zugelement verbunden ist, wobei bevorzugt die Aufnahme proximal und das Zugelement sich distal aus der Aufnahme erstreckt, wobei das Zugelement gelenkig mit der Halteanordnung verbunden ist, wobei bevorzugt die Anbindung des mindestens eines Anbindungselements an die Aufnahme an einem distalen Ende der Aufnahme angeordnet ist. Bei einer Relativbewegung des Auszugskörpers und des daran befestigten Zugelements gegenüber der Aufnahme kann die die Bewegung des Zugelements entsprechend der Geometrie der Innenseite der Aufnahme und des Auszugskörpers sowie der Beschaffenheit, insbesondere der Viskosität, des Dämpfungsmediums gedämpft werden.

Wenn das Dämpfungselement eine Aufnahme in Form eines rohrförmigen ersten Dämpferteils und ein relativ zum ersten Dämpferteil entlang einer Auszugsrichtung, welche sich entlang der Längsachse des rohrförmigen ersten Dämpferteils erstreckt, bewegliches Zugelement in Form eines zweiten Dämpferteils aufweist, wobei sich der zweite Dämpferteil teilweise im Inneren des rohrförmigen ersten Dämpferteils erstreckt und darin den Auszugskörper ausweist und wobei in dem Dämpfungselement ferner ein Dämpfungsmedium enthalten ist, so hat sich dies als besonders bevorzugte Ausführungsform herausgestellt. Bei einer Relativbewegung des zweiten Dämpferteils gegenüber dem ersten Dämpferteil kann die die Bewegung des zweiten Dämpferteils entsprechend der Geometrie der Innenseite des ersten Dämpferteils und des Auszugskörpers des zweiten Dämpferteils sowie der Beschaffenheit, insbesondere der Viskosität, des Dämpfungsmediums gedämpft werden.

Um eine nochmals verbesserte Positionstreue der Stützanordnung bereitzustellen, kann oberhalb des Außenknöchels, bevorzugt am zugsteifen Bereich, ein Volumenkörper, bevorzugt eine Pelotte, zum Abstützen von proximal auf den Außenknöchel angeordnet sein und/oder oberhalb des Innenknöchels ein Volumenkörper, bevorzugt eine Pelotte, zum Abstützen von proximal auf den Innenknöchel angeordnet sein.

Gemäß einer besonders bevorzugten weiteren Ausführungsform ist das Dämpfungselement im Wesentlichen distal des Knöchels angeordnet. Unter distal zum Knöchel ist hier zu verstehen, dass sich das Dämpfungselement im an einen Fuß angelegten beziehungsweise angezogenen Zustand der Fußbewegungsbegrenzungsvorrichtung distal zum Knöchel gelegen befindet.

Gemäß einer weiteren bevorzugten Ausführungsform die die Vorrichtung an einen Strumpf, Socken einer Bandage oder einer Orthese angebunden.

Die oben gestellte Aufgabe wird ferner durch einen Schuh zum Begrenzen einer Fußbewegung über das Sprunggelenk mit den Merkmalen des Anspruchs 13 gelöst. Vorteilhafte Weiterbildungen ergeben sich aus den Unteransprüchen, der Beschreibung und den beigefügten Figuren.

Entsprechend wird ein Schuh zum Begrenzen einer Fußbewegung über das Sprunggelenk vorgeschlagen, umfassend einen Sohlenbereich und einen an den Schuhsohlenbereich angebundenes Schuhoberteil. Erfindungsgemäß weist der Schuh eine Fußbewegungsbegrenzungsvorrichtung gemäß einer der vorstehenden Ausführungsformen auf.

Dadurch dass der Schuh eine Fußbewegungsbegrenzungsvorrichtung gemäß einer der vorstehenden Ausführungsform aufweist, können die zur Fußbewegungsbegrenzungsvorrichtung beschriebenen Vorteile und Wirkungen analog erzielt werden.

Fernerhin ist es dadurch nicht erforderlich, dass eine Person die sein Sprunggelenk beziehungsweise dessen Bandapparat schützen will, eine Fußbewegungsbegrenzungsvorrichtung und zusätzlich einen Schuh anziehen muss, sondern kann die Schutzwirkung dadurch erlangen, dass er einen entsprechend ausgebildeten Schuh, welcher Fußbewegungsbegrenzungsvorrichtung bereits umfasst, anzieht.

Als "Schuh" wird hier jede Form von schuhartiger Fußbekleidung mit einem Oberteil beziehungsweise Schaft und einer damit verbundenen festen Unterlage beziehungsweise Sohle verstanden, insbesondere orthopädische Schuhe, Sportschuhe, Freizeitschuhe und Stiefel oder Sandalen. Das Schuhoberteil und/oder die Sohle können dabei vollflächig ausgebildet sein, oder lediglich Segmente aufweisen, die zur Rückhaltung und Kraftübertragung bei einer Inversion beziehungsweise Supination des Fußes notwendig sind. Der Schuh kann ebenso als Grundstruktur eine Socke oder einen Stumpf aus einem Textilgewebe oder Textilgewirk aufweisen, an welchem beziehungsweise welcher zumindest an Teilbereichen Segmente am Schuhoberteil und/oder an der Sohle angeordnet sind, mittels welchen die zum Rückhalten des Fußes bei einer Inversion beziehungsweise Supination erforderlichen Kräfte aufgenommen und weitergleitet werden können.

Gemäß einer weiter bevorzugten Ausführungsform ist die Halteanordnung in den Sohlenbereich integriert ist und/oder die Stützanordnung in einen Schaftbereich des Schuhs integriert, wobei das Anbindungselement relativ zum Schuhoberteil beweglich ist. Durch die Integration von Stützanordnung und/oder Halteanordnung in den Schuh kann ein besonders kompakter Aufbau des Schuhs erzielt werden. Durch die Bereitstellung der Beweglichkeit des Anbindungselements relativ zum Schuhoberteil ist es insbesondere möglich, dass sich das Anbindungselementbei einer Bewegung des Fußes um das Sprunggelenk, was einer Änderung der relativen Position des Fußes zum Unterschenkel und ebenso zum Knöchel entspricht, relativ zum Schuhoberteil bewegen kann, um so eine die oben beschriebene Orientierung einzunehmen, welche nahe an einer direkten Kraftschlussrichtung, welche bei einer Inversion zwischen Fuß und Unterschenkel auf die Bänder des Sprunggelenks beziehungsweise auf das Sprunggelenk wirkt, gelegen ist. Dadurch ist, wie bereits oben zur Fußbewegungsbegrenzungsvorrichtung beschrieben, die Beweglichkeit des Fußes, mithin der Tragekomfort für die den Schuh tragenden Person erhöht. Zudem ist durch die Möglichkeit, dass sich das Anbindungselementnahe an die beziehungsweise in der direkten Kraftrichtung orientiert, die Schutzwirkung, welche über die Fußbewegungsbegrenzungsvorrichtung bereitgestellt werden kann, im Vergleich herkömmlichen Schuhen erhöht, ohne dabei eine signifikante Verschlechterung der Beweglichkeit über das Sprunggelenk zu bedingen.

Wenn die Fußbewegungsbegrenzungsvorrichtung im Wesentlichen komplett in den Schuh integriert ist, kann zum einen das optische Erscheinungsbild des Schuhs im Vergleich zu einer Ausführungsform ohne Fußbewegungsbegrenzungsvorrichtung im Wesentlichen beibehalten werden. Da Schuhe mehr und mehr aufgrund ihres äußeren Erscheinungsbilds erworben werden, hat dies unter anderem den Vorteil, dass Personen, welchen ein äußeres Erscheinungsbild des Schuhs besonders wichtig ist, ebenso auf einen Schuh mit Fußbewegungsbegrenzungsvorrichtung zurückgreifen können, ohne subjektive optische beziehungsweise ästhetische Einbußen hinnehmen zu müssen.

Zudem kann durch die komplette Integration der Fußbewegungsbegrenzungsvorrichtung in den Schuh ein besonders guter Schutz der Fußbewegungsbegrenzungsvorrichtung, beziehungsweise von dessen Komponenten, bereitgestellt werden. Weiterhin kann auch eine sehr hohe Güte der Positionsgenauigkeit von Stützelement, Halteelement, Anbindungselement und optional Dämpfungselement zum Schuh erzielt werden.

Bei Schuhen, dessen Schuhoberteil im Wesentlichen komplett zugsteife ausgebildet ist, beispielsweise bei Wanderschuhen, Sicherheitsschuhen oder hochschaftigen Sportschuhen, wie etwa Basketballschuhen, kommt es bei einer Inversion aufgrund der dadurch erzeugten Vergrößerung des Abstands vom Vorfuß zum Bereich oberhalb des Knöchels zu einem Nachunten-ziehen des Schaftes am und oberhalb des Knöchels in Richtung des Vorfußes, mithin in distaler Richtung. Dadurch wird zwangsweise eine Verbindung des Schaftes an den Körperbereich proximal des Knöchels gelöst. Gemäß einer weiter bevorzugten Ausführungsform umfasst daher der Schuh, bevorzugt zumindest in einem Schaftbereich des Schuhoberteils unterhalb der Stützanordnung, einen flexiblen Verformungsabschnitt zum Ermöglichen einer Relativbewegung der Stützanordnung relativ zur Halteanordnung. Durch diesen flexiblen Verformungsabschnitt ist es möglich, dass bei einer Bewegung über das Sprunggelenk die Stützanordnung und die Halteanordnung an den jeweiligen Abschnitten der unteren Extremität im Wesentlichen in der vorgegebenen und vorgesehenen Position positioniert und mit diesen verbunden bleiben. Mit anderen Worten reißt beispielsweise bei einer Inversion der Kontakt der Stützanordnung zum Unterschenkel und oberen Knöchelbereich nicht ab, da der flexible Verformungsabschnitt die oben beschriebene Relativbewegung ermöglicht.

Gemäß einer weiter bevorzugten Ausführungsform ist die Tasche fest mit dem Schuhoberteil verbunden und bevorzugt in den Schuhoberteil integriert. Dadurch kann der Bewegungsraum für das Anbindungselement und optional das Dämpfungselement relativ zum Schuhoberteil genau vorgegeben werden. Zudem ist eine einfache Integration der Fußbewegungsbegrenzungsvorrichtung in den Schuh ermöglicht.

Es hat sich als besonders vorteilhaft herausgestellt, wenn das Anbindungselement und/oder optional das Dämpfungselement im Wesentlichen distal des Knöchels angeordnet ist. Dadurch kann unter anderem eine absolute Höhe des Schaftes des Schuhes im Vergleich zu Schuhen, bei welchen beispielsweise ein Dämpfungselement auf Höhe des Knöchels oder gar darüber angeordnet ist, reduziert werden. Beispielsweise ist es so ermöglicht, anstelle eines herkömmlichen, sogenannten "high-cut" Schuhes bei gleicher oder verbesserter Schutzwirkung einen "mid-cut" Schuh bereitzustellen, wobei neben der ästhetisch ansprechenderen Form des letztgenannten Schuhs auch der Tragekomfort erhöht sein kann.

Gemäß einer weiteren bevorzugten Ausführungsform ist ein Fenster, bevorzugt ein Sichtfenster, vorgesehen, derart ausgebildet, dass von außerhalb des Schuhs eine Sicht auf zumindest einen Teil des Anbindungselements und/oder des Dämpfungselements freigegeben ist. Das Fenster kann beispielsweise zur visuellen Kontrolle des Anbindungselements und/oder des Dämpfungselements durch eine Person verwendbar sein, oder gar einen Wechsel oder Wartung zumindest von Teilen des Anbindungselements und/oder des Dämpfungselements erleichtern. Das Fenster kann zumindest teilweise ein transparentes Material aufweisen und/oder einen Bereich aufweisen, in dem gar kein Material vorgesehen ist.

Bevorzugt ist dabei das Dämpfungselement derart ausgebildet, dass das Wirkprinzip des Dämpfungselements einsehbar ist. Bevorzugt weist die Aufnahme des Dämpfungselements einen transparenten Bereich auf, so dass eine Sicht in das Innere der Aufnahme bereitgestellt ist.

Das Fenster kann bevorzugt ein Außenmaterial des Schuhs, bevorzugt des Schuhoberteils, einen Bereich der Tasche und/oder einen Bereich der Umhüllung umfassen. Mit anderen Worten können das Außenmaterial des Schuhs, der Bereich der Tasche und/oder der Bereich der Umhüllung ein Fenster beziehungsweise eine Öffnung aufweisen.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch eine perspektivische Seitenansicht einer Fußbewegungsbegrenzungsvorrichtung zum Begrenzen einer Fußbewegung über das Sprunggelenk gemäß einer ersten Ausführungsform;
- Figur 2: schematisch eine perspektivische Seitenansicht einer Fußbewegungsbegrenzungsvorrichtung gemäß einer weiteren Ausführungsform;
- Figur 3: schematisch eine perspektivische Seitenansicht einer Fußbewegungsbegrenzungsvorrichtung gemäß einer weiteren Ausführungsform;
- Figur 4: schematisch eine perspektivische Seitenansicht eines erfindungsgemäßen Schuhs zum Begrenzen einer Fußbewegung über das Sprunggelenk;
- Figur 5: schematisch eine weitere perspektivische Seitenansicht des Schuhs aus Figur 4;
- Figur 6: schematisch eine perspektivische Seitenansicht eines Schuhs gemäß einer weiteren Ausführungsform;
- Figuren 7 bis 11: schematisch weitere Ausführungsformen eines Schuhs zum Begrenzen einer Fußbewegung über das Sprunggelenk;
- Figur 12: schematisch eine weitere Ausführungsform eines Schuhs zum Begrenzen einer Fußbewegung über das Sprunggelenk, bei welchem ein Sichtfenster in dem Schuhoberteil vorgesehen ist;
- Figur 13: schematisch eine perspektivische Seitenansicht einer Fußbewegungsbegrenzungsvorrichtung gemäß einer weiteren Ausführungsform; und
- Figur 14: schematisch eine perspektivische Seitenansicht einer Fußbewegungsbegrenzungsvorrichtung gemäß einer weiteren Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

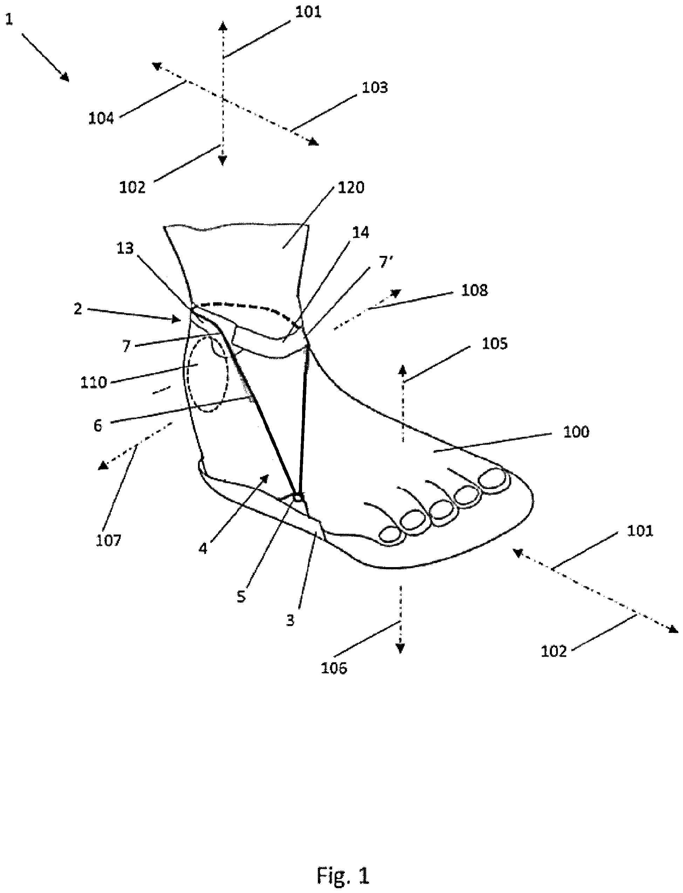

In Figur 1 ist schematisch eine perspektivische Seitenansicht einer Fußbewegungsbegrenzungsvorrichtung 1 zum Begrenzen einer Fußbewegung über das Sprunggelenk gemäß einer ersten Ausführungsform gezeigt, welcher an einem Fuß 100 angebracht ist.

Zum besseren Verständnis sind mittels der Bezugszeichen 101-108 die anatomischen Richtungen in Bezug auf das Sprunggelenk, welche im Folgenden für die Beschreibung der Fußbewegungsbegrenzungsvorrichtung 1 analog gelten, dargestellt. Entsprechend bedeuten die Bezugszeichen 101 proximal, 102 distal, 103 posterior, 104 anterior, 105 dorsal, 106 plantar, 107 lateral und 108 medial.

Die Fußbewegungsbegrenzungsvorrichtung 1 weist eine Stützanordnung 2 zum Abstützen von proximal 101 am Knöchel 110 des Sprunggelenks auf. Ferner weist sie eine Halteanordnung 3 zum Halten an einem Sohlenbereich des Fußes 100 auf. Zwischen der Stützanordnung 2 und der Halteanordnung 3 ist ein Anbindungselement 6angeordnet, mittels welchem eine Relativbewegung zwischen der Stützanordnung 2 und der Halteanordnung 3 begrenzt werden kann.

Das Anbindungselement 6 ist an seinem distalen Ende über eine gelenkige Halterung 5 gelenkig an der Halteanordnung 3 gehalten. Folglich ist das Anbindungselement 6über die gelenkige Halterung 5 um diese herum relativ zur Halteanordnung 3 somit auch zu Fuß 100 beweglich. Mit anderen Worten ist das Anbindungselement 6 an der Halterung 5 verschiebbar gelagert.

Gemäß Figur 1 ist das Anbindungselement 6 als zugsteifes Band ausgebildet und an der Stützanordnung 2 angebunden. Das Anbindungselement 6 erstreckt sich von einer ersten Anbindungsstelle 7 von der Stützanordnung 2 über die Halterung 5 zu einer zweiten Anbindungsstelle 7', an welche es wieder an die Stützanordnung 2 angebunden ist.

Ändert sich eine Stellung des Fußes 100 relativ zum Unterschenkel 120 durch eine Bewegung über das Sprunggelenk, so kann sich das Anbindungselement 6aufgrund der bereitgestellten freien Beweglichkeit relativ zur Halteanordnung 3, insbesondere über die verschiebbare Anbindung an der Halterung 5 relativ zur Stützanordnung 2 entsprechend der Änderung der Fußstellung neu ausrichten.

Die Stützanordnung 2 weist einen zugsteifen Bereich 13 auf, welcher sich von der lateralen Seite ab der ersten Anbindungsstelle 7 posterior herum, über einen Versbereich des Fußes 100, auf die mediale Seite zur zweiten Anbindungsstelle 7' erstreckt. Die Anbindungsstellen 7, 7' sind derart positioniert, dass eine infolge einer Fußbewegung über das Sprunggelenk an der Halterung 5 wirkende Rückhaltekraft über das Anbindungselement 6 in eine lateral-proximal wirkende Komponente und eine medial-proximal wirkende Komponente aufgeteilt wird.

Anterior 103 erstreckt sich zwischen den beiden Anbindungsstellen 7, 7' ein zugelastischer Bereich 14. Der zugelastische Bereich 14 ist derart ausgebildet, dass dieser bei angelegter Fußbewegungsbegrenzungsvorrichtung 1 an den Fuß 100 eine vorgegebene Vorspannung aufweist, wodurch die Stützanordnung 2 relativ zum Knöchel 110 in Position gehalten werden kann. Weiterhin erleichtert der zugelastische Bereich 14 das Vorspannen des Anbindungselements 6 und erlaubt eine Anpassung an verschiedene Knöchelumfänge. Zudem ist ein Einstieg in die Stützanordnung 2 erleichtert.

Ferner bewirkt die Vorspannung des zugelastischen Bereichs 14, dass die Steifigkeit der Stützanordnung 2 erhöht werden kann.

Die gelenkigen Halterung 5 ist hier durch ein Band und eine optionale Öse, welche auf dem Band aufgefädelt ist, ausgebildet, wobei das Band an zwei voneinander beabstandeten Stellen an der Halteanordnung 3 befestigt ist und dadurch eine Schlaufe ausbildet. Das Anbindungselement 6 ist hierbei über die Öse, durch welche das Band verläuft, an dem Band und somit an der Halteanordnung 3 gehalten. Dadurch kann sich das Anbindungselement 6 zum einen zumindest teilweise entlang des Bandes relativ zur Halteanordnung 3 verschieben. Ferne ist es möglich, dass das Anbindungselement 6 relativ zur Halteanordnung 3 verschwenkt werden kann.

Das Band ist hierbei im Vergleich zur Größe der Vorrichtung derart kurz, dass die gelenkige Halterung 5 im Wesentlichen an einer festen Stelle der Halteanordnung 3 vorliegt. Hierdurch ist sichergestellt, dass die Krafteinleitung in die Halteanordnung 3 auf Höhe einer bestimmten Position relativ zum Fuß 100, bevorzugt in einem Grenzbereich zwischen Mittelfuß und Vorfuß, und weiter in den Fuß 100 erfolgt.

Alternativ kann die das Band auch als Öse fungieren, wobei das Band eine Schlaufe bereitstellt, durch welle das Anbindungselement 6 verläuft und durch welche es umgelenkt wird. Ferner alternativ kann die Halterung 5 auch in Form einer starren Öse, welche an der Halteanordnung 3 angebracht ist, bereitgestellt sein.

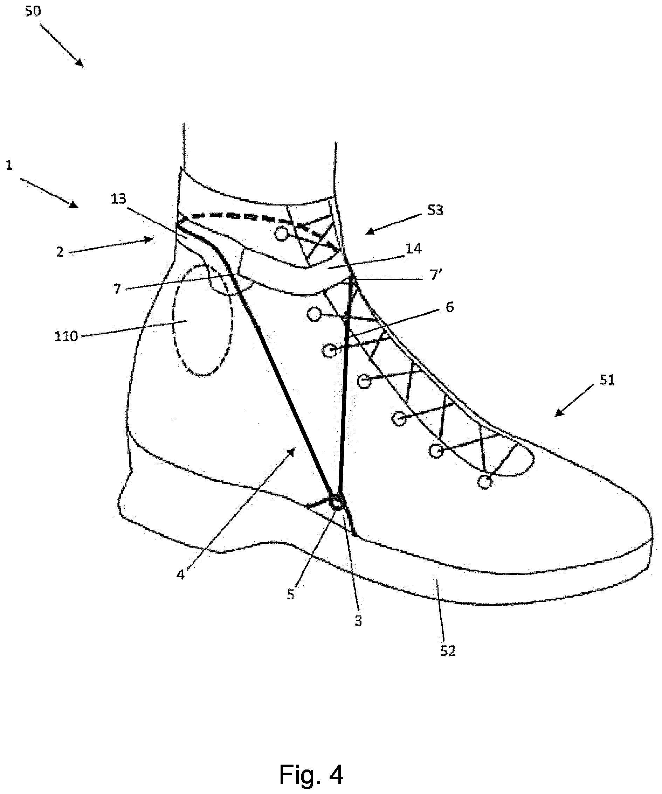

Die Fußbewegungsbegrenzungsvorrichtung 1 gemäß Figur 1 kann als separate Vorrichtung in einem herkömmlichen Schuh getragen werden. Hierzu kann die Halteanordnung 3 beispielsweise unter eine herausnennbare Innensohle des Schuhs gelegt werden und die Stützanordnung 2 beim Anziehen des Schuhs an die vorgegebene Position oberhalb des Knöchels 110 angebracht werden. Alternativ kann die Fußbewegungsbegrenzungsvorrichtung gemäß dem in Figur 1 dargestellten Prinzip beispielsweise in einen Schuh zum Begrenzen einer Fußbewegung über das Sprunggelenk integriert werden, wie etwa in Figur 4 gezeigt.

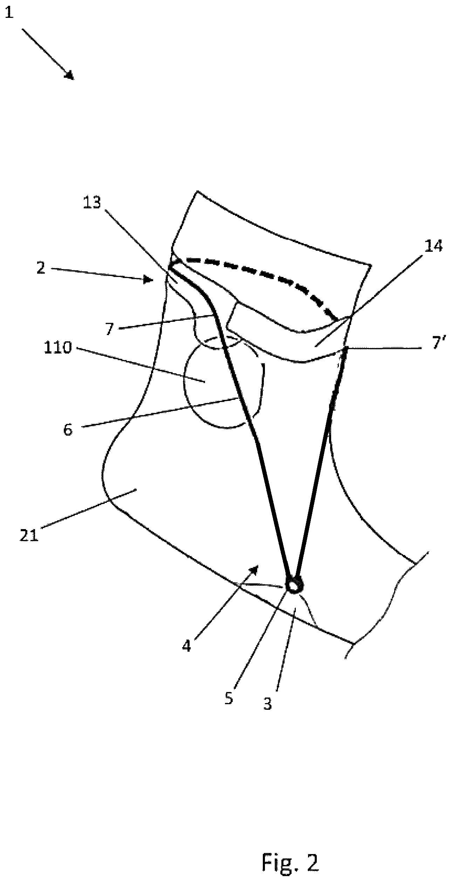

Figur 2 zeigt schematisch eine perspektivische Seitenansicht einer Fußbewegungsbegrenzungsvorrichtung 1 gemäß einer weiteren Ausführungsform. Die hier gezeigte Fußbewegungsbegrenzungsvorrichtung 1 entspricht in ihrem Aufbau im Wesentlichen der zu Figur 1 beschriebenen, wobei als Grundstruktur ein elastischer Socken 21 vorgesehen ist, an welchem sowohl die Halteanordnung 3 als auch die Stützanordnung 2 angebracht sind. Der elastische Socken 21 kann beispielsweise in Form einer herkömmlichen Bandage bereitgestellt sein. Wiederum ist das Anbindungselement 6 durch die gelenkige Halterung 5 gelenkig an der Halteanordnung 3 gehalten und wie oben beschrieben über die Anbindungsbereiche 7, 7' an der Stützanordnung 2 angebunden. Vorteilhaft an dieser Ausführungsform ist, dass die Fußbewegungsbegrenzungsvorrichtung 1 durch ein einfaches Überstreifen des Sockens 21 über den Fuß 100 angelegt werden kann. Danach kann der Fuß beispielsweise mit einem herkömmlichen Schuh versehen werden.

Zudem ist hier die gelenkige Halterung 5 als Drehgelenk ausgebildet und an einer festen Stelle der Halteanordnung 3 angeordnet. Das Anbindungselement 6 ist relativ zur Halteanordnung um eine hier nicht gezeigte Schwenkachse schwenkbar an der Halteanordnung 3 angebunden beziehungsweise gehalten.

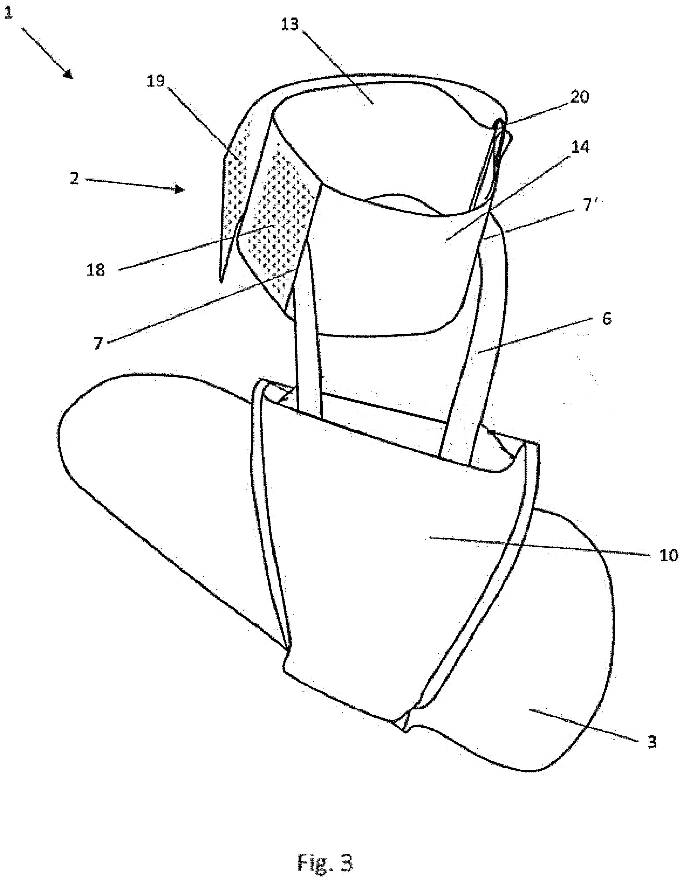

Figur 3 zeigt schematisch eine perspektivische Seitenansicht einer Fußbewegungsbegrenzungsvorrichtung 1 gemäß einer weiteren Ausführungsform, welche beispielsweise wie die zu Figur 1 beschriebene Vorrichtung separat in einem herkömmlichen Schuh getragen werden kann oder aber in einen Schuh zum Begrenzen einer Fußbewegung über das Sprunggelenk zum Bereitstellen der vorgenannten Wirkung in diesen zumindest teilweise integriert werden kann.

Die Fußbewegungsbegrenzungsvorrichtung 1 gemäß dieser Ausführungsform weist eine sohlenartige Halteanordnung 3 auf. Die Halteanordnung 3 kann sich dabei im Wesentlichen über den gesamten Sohlenbereich oder nur über einen Teil des Sohlenbereichs des Fußes erstrecken.

Die Stützanordnung 2 weist wiederum einen zugsteifen Bereich 13 und einen zugelastischen Bereich 14 auf. Gemäß dieser Ausführungsform ist der zugsteife Bereich 13 in Form einer zugsteifen Schlaufe ausgebildet, welche durch eine Öse 20 geführt und in dieser umgelenkt ist. Am Ende der Schlaufe ist ein Haftbereich 19 ausgebildet, welcher mit einem entsprechenden gegenüberliegenden Haftbereich 18 wechselwirken kann, um eine Fixierung des Schlaufenendes am körpernahen Bereich zu ermöglichen. Die Haftbereiche 18, 19 können beispielsweise als Klettverschluss ausgebildet sein. Der sich anterior erstreckende zugelastische Bereich 14 ist mit dem zugfesten Bereich 13 an einem Ende vernäht und am anderen Ende über die Öse 20 mit diesem verbunden.

Zwischen der Stützanordnung 2 und der Halteanordnung 3 ist wiederum ein Anbindungselement 6 vorgesehen, welches wie Figuren 1 und 2 beschrieben über eine gelenkige Halterung 5 (in dieser Figur nicht gezeigt) gelenkig an der Halteanordnung 3 gehalten ist.

Mit der Halteanordnung 3 ist eine sich medial erstreckende Tasche 10 verbunden, in welcher das Anbindungselement 6 teilweise aufgenommen ist. Die Tasche 10 stellt dabei einen Bewegungsraum für das Anbindungselement 6 bereit, in welchem es sich frei um die bewegliche Halterung 5 bewegen kann.

Sowohl die Tasche 10 als auch das Anbindungselement 6 sind jeweils aus einem Material, vorliegend einem Gewebe, ausgebildet, welches jeweils einen geringen Reibungskoeffizienten aufweist, wobei optional zwischen der Tasche 10 und dem Anbindungselement 6 ein Reibungskoeffizient von vorzugsweise kleiner gleich 0,5, bevorzugt kleiner 0,2 und besonders bevorzugt kleiner 0,1 vorliegt. Dadurch kann eine leichtgängige Bewegung des Anbindungselements 6 in der Tasche 10 ermöglicht werden. Alternativ kann auch das Material der Tasche 10 und/oder das Material des Anbindungselements 6 zumindest teilweise mit einer reibungsarmen Beschichtung versehen sein.

Vorliegend ist die Rückseite der Tasche 10, das heißt die körpernahe, innere mediale Seite, einstückig mit der Halteanordnung 3 aus einem zugsteifen Gewebe ausgebildet. Die Vorderseite der Tasche 10, das heißt die laterale Seite, ist durch Aufnähen eines weiteren Gewebeteils realisiert. Alternativ können die Tasche 10 und die Halteanordnung 3 auch anderweitig ausgebildet sein, beispielsweise als separate Komponenten. Die Halteanordnung 3 und die Tasche 10 sind dabei nur optional miteinander verbunden, sie können ebenso auch nicht miteinander verbunden sein.

Aus Figur 4 ist schematisch eine perspektivische Seitenansicht eines erfindungsgemäßen Schuhs 50 zum Begrenzen einer Fußbewegung über das Sprunggelenk gezeigt. Der Schuh 50 weist einen Sohlenbereich 52 und einen an dem Sohlenbereich 52 angebundenen Schuhoberteil 51 auf. Zudem umfasst der Schuh 50 eine Fußbewegungsbegrenzungsvorrichtung 1, welche im Wesentlichen analog zu der Ausführungsform zu Figur 1 beschrieben ausgebildet ist.

Die Halteanordnung 3 ist hierbei in den Sohlenbereich 52 integriert. Die Stützanordnung 2 ist in einem Schaftbereich 53 des Schuhs 50 integriert. Die Stützanordnung 2 weist an ihren Enden die Haltebereiche 7 und 7' auf. Das Anbindungselement 6 erstreckt sich von der lateral angeordneten, ersten Anbindungsstelle 7 über die Halterung 5 zur medial angeordneten, zweiten Anbindungsstelle 7'.Dabei ist das Anbindungselement 6 über die gelenkige Halterung 5 relativ zur Halteanordnung 3 bzw. zum Sohlenbereich 52 beweglich angeordnet. Aufgrund der beweglichen Anbindung des Anbindungselements 6 zur Halteanordnung 3 ist das Anbindungselement 6 relativ zum Schuhoberteil 51 beweglich.

Bei dem Schuh 50 gemäß dieser Ausführungsform ist das Anbindungselement 6 großteils distal des Knöchels 110 angeordnet.

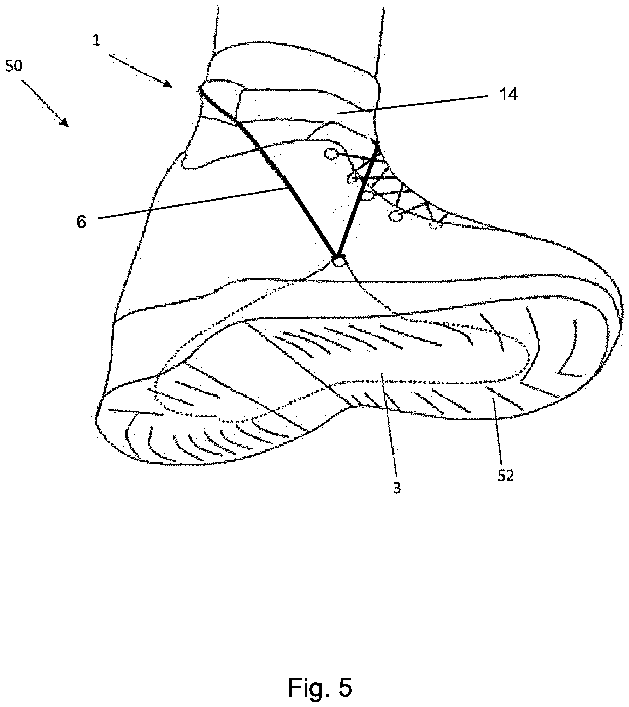

In Figur 5 ist schematisch eine weitere perspektivische Seitenansicht des Schuhs 50 aus Figur 4 gezeigt. Hierbei ist die Anordnung der Halteanordnung 3 im Sohlenbereich 52 mittels einer gestrichelten Linie angedeutet.

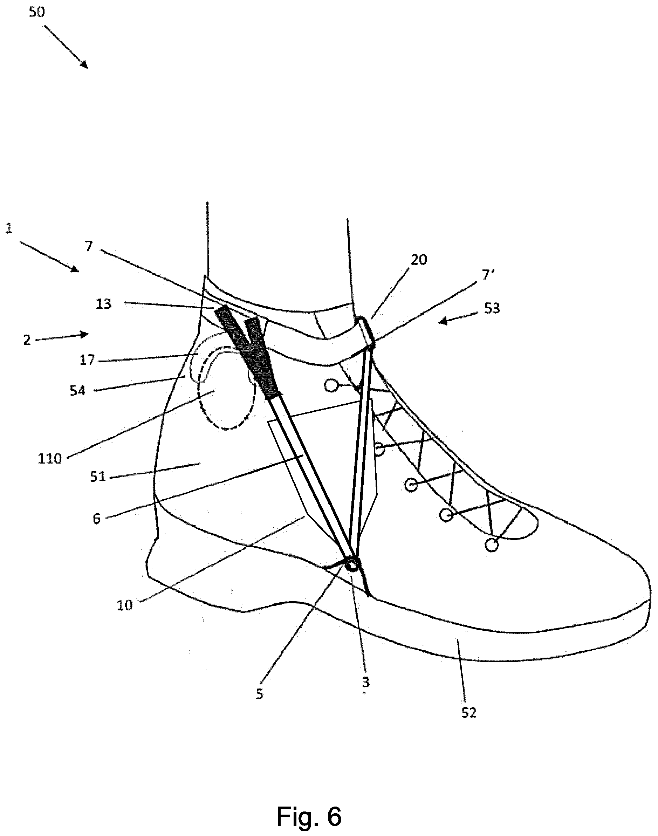

In Figur 6 ist schematisch eine perspektivische Seitenansicht eines Schuhs 50 gemäß einer weiteren Ausführungsform gezeigt. Der Aufbau des Schuhs 50 entspricht im Wesentlichen jenem, welcher im Hinblick auf Figur 4 beschrieben wurde. Zusätzlich umfasst der Schuh 50 gemäß Figur 6 eine Tasche 10 ähnlich der in Figur 3 beschrieben, wobei die Tasche aus Gründen der verbesserten Veranschaulichung offen dargestellt ist.

Die Tasche 10 ist fest mit dem Schuhoberteil 51 verbunden. Sie stellt wiederum einen Bewegungsraum für das Anbindungselement 6 bereit. Das Anbindungselement 6 ist dabei in den Grenzen des Bewegungsraums relativ zum Schuhoberteil 51 beweglich angeordnet.

Ferner ist der Stützbereich 2 im Wesentlichen wie zu Figur 3 beschrieben ausgebildet. Der zugfeste Bereich 13 der Stützanordnung 2 kann mithin über ein Anziehen über die Öse 20 oberhalb des Knöchels 110 am Körper der den Schuh 50 tragenden Person vorgespannt angebracht werden. Zusätzlich ist eine Pelotte 17 vorgesehen, welche zum einen dazu beiträgt, die Stützanordnung 2 an der für sie vorgesehenen Position in Bezug auf den Körper der den Schuh 50 tragenden Person zu halten. Zudem kann über die Pelotte 17 ein verbessertes Abstützen von proximal auf den Knöchel 110 erzielt werden.

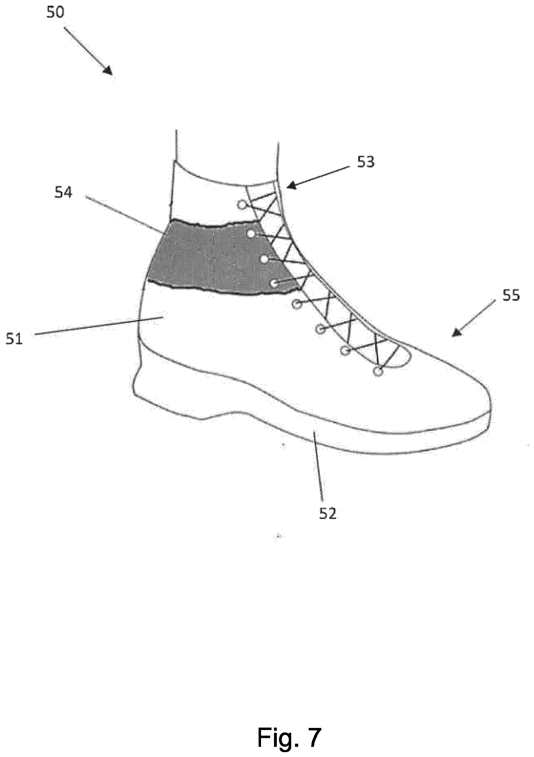

Um eine Relativbewegung der Stützanordnung 2 relativ zur Halteanordnung 3 bereitzustellen, weisen die vorgeschriebenen Schuhe 50 optional einen flexiblen Verformungsabschnitt auf. In den Figuren 7 bis 11 sind schematisch verschiedene Ausführungsformen von Schuhen 50 mit flexiblen Verformungsabschnitten 54 gezeigt, wobei aus Gründen der besseren Übersichtlichkeit die darin integrierten beziehungsweise die daran angeordneten Fußbewegungsbegrenzungsvorrichtungen 1 nicht dargestellt sind.

Gemäß der beispielhaften Ausführungsform des Schuhs 50 gemäß Figur 7 erstreckt sich der flexible Verformungsabschnitt 54 umfänglich im Wesentlichen im Gesamten zwischen dem Schaft 53 und einem Fußbereich 55 des Schuhoberteils 51. Dadurch ist eine besonders hohe Beweglichkeit der am Schaft 53 angeordneten Stützanordnung 2 relativ zur im Sohlenbereich 52 angeordneten Halteanordnung 3 bereitgestellt. Bevorzugt sind die übrigen Bereiche des Schuhoberteils 51 im Wesentlichen analog zu herkömmlichen Ausführungen steif, bevorzugt zugsteif, ausgebildet.

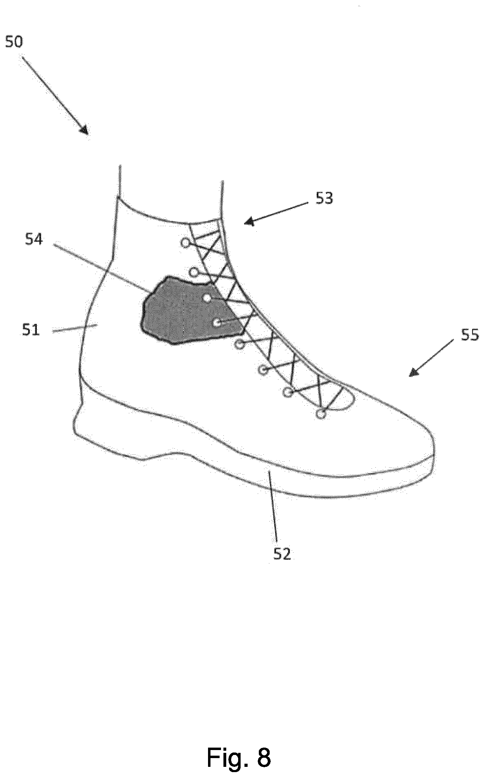

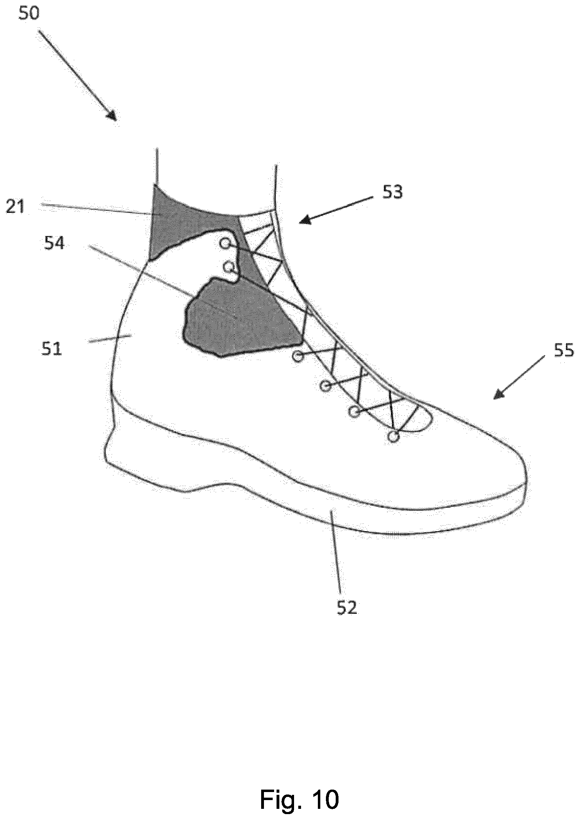

In Figur 8 ist schematisch eine weitere Ausführungsform eines Schuhs 50 gezeigt. Der flexible Verformungsabschnitt 54 erstreckt sich anders als in Figur 10 gezeigt nur in einem Teilbereich, wobei der flexiblen Verformungsabschnitt 54 derart angeordnet ist, dass dennoch eine Relativbewegung der Stützanordnung 2 relativ zur Halteanordnung 3 gegeben ist. Durch diese Ausbildung ist zusätzlich erzielt, dass der Schaftbereich 53 sich über den Fersenbereich des Schuhoberteils 51 aufgrund des dortigen Vorsehens einer relativ steifen Struktur des Schuhoberteils 51 zusätzlich gegenüber dem Sohlenbereich 52 Abstützen kann.

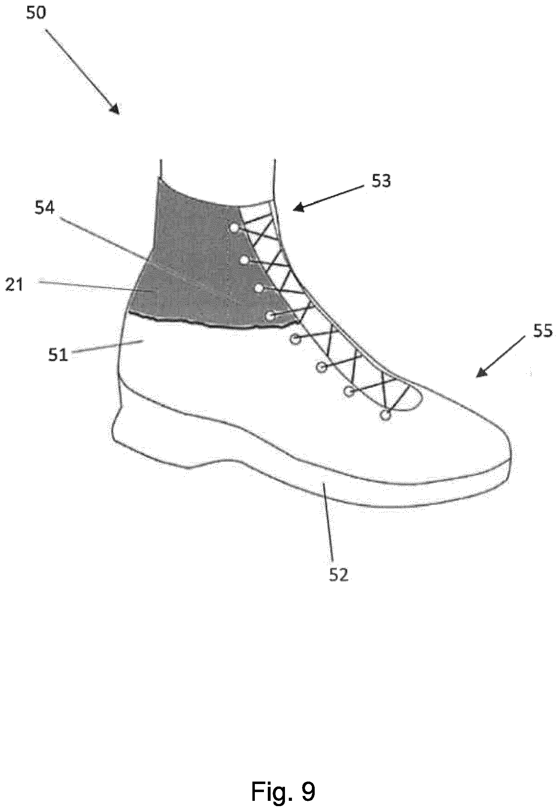

Figur 9 zeigt schematisch eine perspektivische Seitenansicht eines Schuhs 50 gemäß einer weiteren Ausführungsform. Hierbei ist der gesamte obere Abschnitt inklusive des kompletten Schaftes 53 flexibel ausgebildet. Hierdurch ist ein besonders hoher Tragekomfort gegeben. Die Stützanordnung 2 ist bei Schuhen 50 gemäß dieser beispielhaften Ausbildung bevorzugt wie zu Figur 3 beschrieben ausgebildet. Der flexible Bereich 54 kann vorliegend über einen Sockenbereich realisiert sein, welcher als Teil des Schuhs 50 ausgebildet ist und mit welchem der steife Bereich des Fußbereichs 55 des Schuhoberteils 51 unterhalb des Knöchels 110 verbunden ist. Alternativ kann sich der Sockenbereich auch im Wesentlichen über den gesamten Schuh 50 erstrecken, und/oder als sockenförmiger Innenschuh ausgebildet sein.

Der in Figur 10 schematisch dargestellte Schuh 50 ist im Wesentlichen wie der Schuh in Figur 8 ausgebildet, wobei sich der sensible Verformungsabschnitt 54 zusätzlich über Teile des Schaftes 53 erstreckt, wodurch der Tragekomfort abermals erhöht ist.

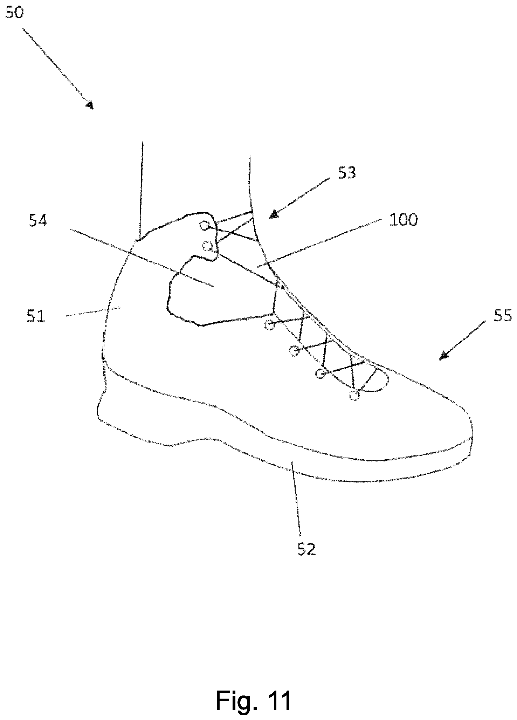

Wie schematisch in Figur 11 dargestellt, kann optional ein Schuh 50 auch einen flexiblen Verformungsabschnitt 54 in Form einer im Wesentlichen kompletten Materialaussparung in diesem Bereich aufweisen.

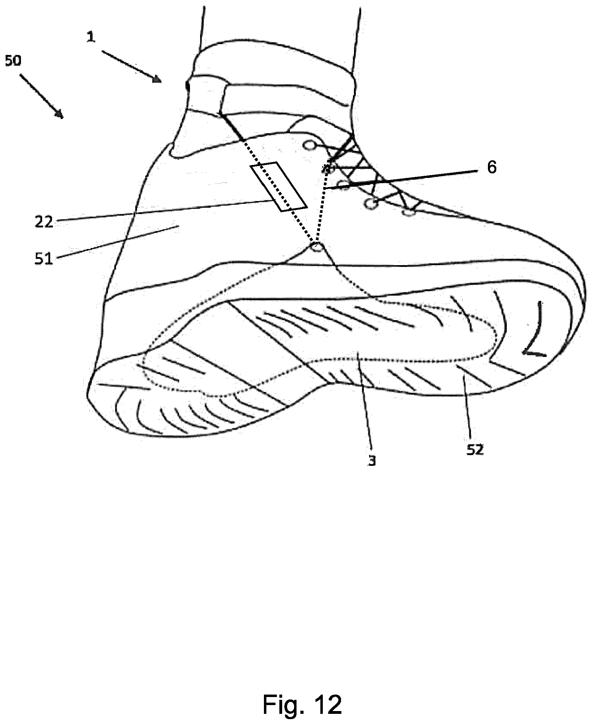

Figur 12 zeigt schematisch eine weitere Ausführungsform eines Schuhs 50 zum Begrenzen einer Fußbewegung über das Sprunggelenk. Der Schuh 50 ist im Wesentlichen gemäß der in den Figuren 4 und 5 gezeigten Ausführungsform ausgebildet. Das Anbindungselement 6 ist hierbei jedoch zusätzlich in den Schuh 50 integriert. Mit anderen Worten ist das Anbindungselement 6 unterhalb einer äußeren Decklage des Schuhoberteils 51 innerhalb des Schuhs angeordnet, so dass das Anbindungselement 6 von außen nicht sichtbar ist. Zusätzlich ist ein optionales Sichtfenster 22 in dem Schuhoberteil 50 vorgesehen, durch welches von außen eine freie Sicht auf einen Teil des Anbindungselement 6 gegeben ist. Zum besseren Verständnis ist der abgedeckte Teil des Anbindungselement 6 mittels gestrichelter Linien angedeutet.

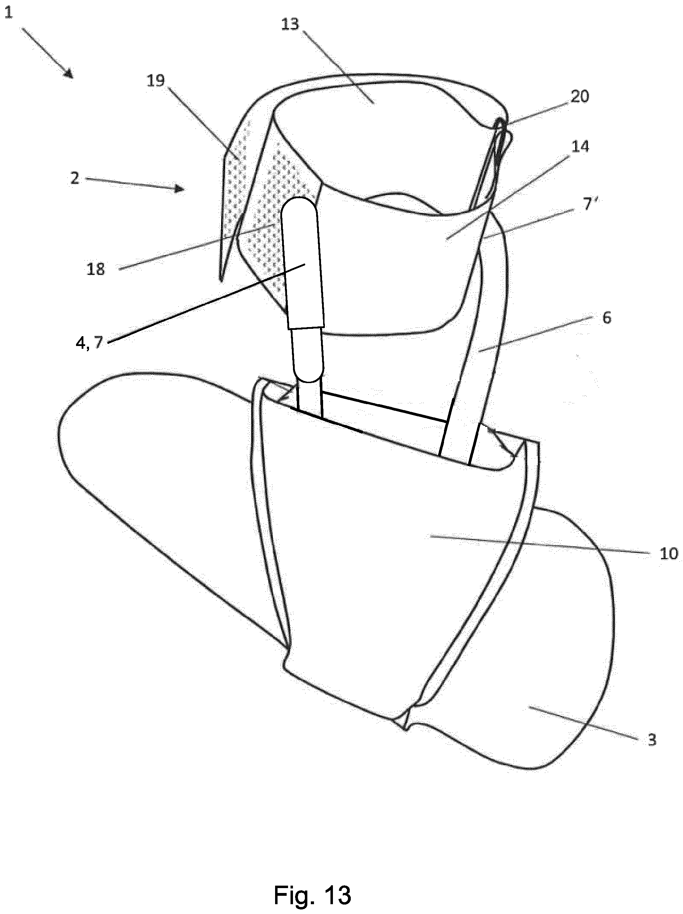

Figur 13 zeigt schematisch eine perspektivische Seitenansicht einer Fußbewegungsbegrenzungsvorrichtung 1 gemäß einer weiteren Ausführungsform, welche im Wesentlichen jener aus Figur 3 entspricht. Mithin ist der zugsteife Bereich 13 der Stützanordnung 2 als Schlaufe ausgebildet und der zugelastische Bereich 14 an einem Ende fest mit dem zugelastischen Bereich 13 verbunden und weist am anderen Ende die Öse 20 auf, durch welche die Schlaufe fädelbar ist.

Ferner ist das Anbindungselement 6 über ein Dämpfungselement 4 an der Stützanordnung 2 gehalten. Da das Dämpfungselement 4 direkt an dem zugsteifen Bereich 13 angebracht ist, stellt das Dämpfungselement 4 ebenfalls den ersten Anbindungsbereich 7 dar.

Alternativ kann der zweite Anbindungsbereich 7' auch im Bereich der Öse 20 auf Seiten des zugelastischen Bereichs 14 angeordnet sein, wobei die Öse 20 dann einen Teil des Anbindungsbereichs 7' darstellt.

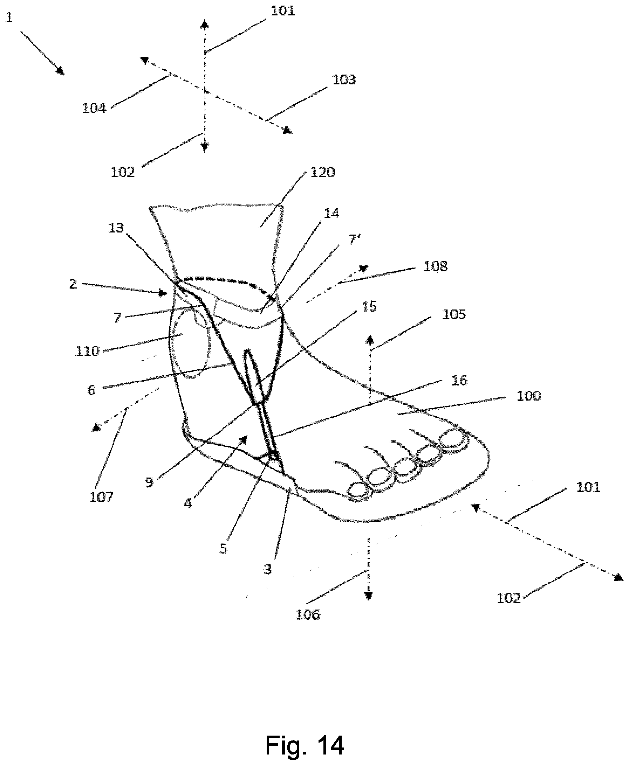

Figur 14 zeigt schematisch eine perspektivische Seitenansicht einer Fußbewegungsbegrenzungsvorrichtung 1 gemäß einer weiteren Ausführungsform, welche im Wesentlichen jener aus Figur 1 entspricht. Bei dieser Ausführung ist das Anbindungselement 6 an über ein Dämpfungselement 4 an der gelenkigen Halterung 5 gehalten.

Das Dämpfungselement 4 ist an seinem distalen Ende an der gelenkigen Halterung 5 gelenkig an der Halteanordnung 3 gehalten. Folglich ist das Dämpfungselement 4 und dadurch das Anbindungselement 6 über die gelenkige Halterung 5 um diese herum relativ zur Halteanordnung 3 somit auch zu Fuß 100 beweglich.

Das Anbindungselement 6 erstreckt sich von einer ersten Anbindungsstelle 7 von der Stützanordnung 2 über eine Anbindung 9 an das Dämpfungselement 4 zu einer zweiten Anbindungsstelle 7', an welche es wieder an die Stützanordnung 2 angebunden ist. An der Anbindung 9 ist das Anbindungselement 6 verschiebbar mit dem Dämpfungselement 4 verbunden. Mit anderen Worten kann sich das Dämpfungselement 4 über die Anbindung 9 entlang des Anbindungselements 6 verschieben.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezuqszeichenliste

- 1: Fußbewegungsbegrenzungsvorrichtung
- 2: Stützanordnung
- 3: Halteanordnung
- 4: Dämpfungselement
- 5: Gelenkige Halterung
- 6, 6': Anbindungselement
- 7, 7': Anbindungsstelle
- 8: Mittelspline
- 9, 9': Anbindung
- 10: Tasche
- 11: Umhüllung
- 12: Führungsöffnung
- 13: Zugsteifer Bereich
- 14: Zugelastischer Bereich
- 15: Aufnahme
- 16: Zugelement
- 17: Pelotte
- 18: Haftbereich
- 19: Haftbereich
- 20: Öse
- 21: Socke
- 22: Sichtfenster

- 50: Schuh
- 51: Schuhoberteil
- 52: Sohlenbereich
- 53: Schaftbereich
- 54: Flexibler Verformungsabschnitt
- 55: Fußbereich

- 100: Fuß
- 101: proximal
- 102: distal
- 103: posterior
- 104: anterior
- 105: dorsal
- 106: plantar
- 107: lateral
- 108: medial
- 110: Knöchel

## Patentansprüche

1. Fußbewegungsbegrenzungsvorrichtung (1) zum Begrenzen einer Fußbewegung über das Sprunggelenk, umfassend eine Stützanordnung (2) zum Abstützen an einem Unterschenkel oder von proximal am Sprunggelenk-Knöchel (110), eine Halteanordnung (3) zum Halten an einem Fuß (100), bevorzugt einem Fußsohlenbereich, und mindestens ein Anbindungselement (6) zum Begrenzen einer Relativbewegung zwischen der Stützanordnung (2) und der Halteanordnung (3),
wobei das mindestens eine Anbindungselement (6) an zwei verschiedenen Anbindungsstellen (7, 7') an die Stützanordnung (2) angebunden ist, wobei die Stützanordnung (2) einen sich von einer lateralen Seite von der ersten Anbindungsstelle (7) posterior herum zumindest auf eine mediale Seite zur zweiten Anbindungsstelle (7') erstreckenden, zugsteifen Bereich (13) umfasst, oder die Stützanordnung (2) einen sich von einer lateralen Seite von der ersten Anbindungsstelle (7) anterior herum zumindest auf eine mediale Seite zur zweiten Anbindungsstelle (7') erstreckenden, zugsteifen Bereich (13) umfasst,
wobei der zugsteife Bereich (13) an seinen Enden durch einen zugelastischen Bereich (14) verbunden ist, wobei sich der zugelastische Bereich (14) zwischen der ersten Anbindungsstelle (7) und der zweiten Anbindungsstelle (7') entgegengesetzt zum zugsteifen Bereich (13) erstreckt
**dadurch gekennzeichnet, dass**
das mindestens eine Anbindungselement (6) an einem Punkt zwischen den beiden Anbindungsstellen (7, 7') über eine gelenkige Halterung (5) an der Halteanordnung (3) gehalten ist,
wobei die gelenkige Halterung (5) derart ausgebildet ist, dass das Anbindungselement (6) in einem vorgegebenen Bereich verschiebbar zur Halteanordnung (3) an diese angebunden ist.

2. Fußbewegungsbegrenzungsvorrichtung (1) gemäß Anspruch 1, wobei das mindestens eine Anbindungselement (6) an zwei verschiedenen Anbindungsstellen (7, 7') an der Stützanordnung (2) angebunden ist, wobei sich das zumindest eine Anbindungselement (6) bevorzugt von einer Anbindungsstelle (7) zur anderen Anbindungsstelle (7') erstreckt und das mindestens eine Anbindungselement (6) zwischen den beiden Anbindungsstellen (7, 7') verschiebbar an der Halteanordnung (3) gehalten ist und/oder die Anbindungsstellen (7, 7') relativ zum Anbindungselement (6) derart positioniert sind, dass eine infolge einer Fußbewegung über das Sprunggelenk im Anbindungselement (6) entstehende Rückhaltekraft in eine lateral-proximal wirkende Komponente und eine medial-proximal wirkende Komponente aufgeteilt wird.

3. Fußbewegungsbegrenzungsvorrichtung (1) gemäß Anspruch 1 oder 2, wobei das mindestens eine Anbindungselement (6) über ein Dämpfungselement (4) an der Stützanordnung (2) oder der Halteanordnung (3) gehalten ist, wobei bevorzugt das Dämpfungselement (4) an dem zugsteifen Bereich (13) der Stützanordnung (2) befestigt ist.

4. Fußbewegungsbegrenzungsvorrichtung (1) gemäß Anspruch 3, wobei das Dämpfungselement (4) am zumindest einen Anbindungselement (6) verschiebbar gehalten ist, wobei das Dämpfungselement (4) zwischen den beiden Anbindungsstellen (7, 7') an das Anbindungselement (6) angebunden ist.

5. Fußbewegungsbegrenzungsvorrichtung (1) gemäß Anspruch 3 oder 4, ferner umfassend ein zweites Anbindungselement (6'), wobei bevorzugt
das erste Anbindungselement (6) bezogen auf einen Mittelspline (8), bevorzugt eine Mittelachse, des Dämpfungselements (4) beabstandet von dem Mittelspline (8), bevorzugt der Mittelachse, auf einer ersten Seite des Dämpfungselements (4) bevorzugt gleitend an das Dämpfungselement (4) angebunden ist, und
das zweite Anbindungselement (6') bezogen auf den Mittelspline (8), bevorzugt die Mittelachse, des Dämpfungselements (4) beabstandet von dem Mittelspline (8), bevorzugt der Mittelachse, auf einer zweiten Seite des Dämpfungselements (4) bevorzugt gleitend an das Dämpfungselement (4) angebunden ist,
wobei bevorzugt der Abstand der Anbindungen (9, 9') von erstem Anbindungselement (6) und zweitem Anbindungselement (6') zum Dämpfungselement (4) bezogen zum Mittelspline (8), bevorzugt zur Mittelachse, im Wesentlichen gleich groß ist,
wobei bevorzugt das erste Anbindungselement (6) und das zweite Anbindungselement (6') gemeinsam als Einzelteil ausgebildet sind und das Einzelteil an der ersten Anbindungsstelle (7) oder der zweiten Anbindungsstelle (7') umgelenkt und gleitend geführt ist.

6. Fußbewegungsbegrenzungsvorrichtung (1) gemäß einem der Ansprüche 3 - 5, wobei eine Tasche (10) zum zumindest teilweisen Aufnehmen des Anbindungselements (6) und/oder des Dämpfungselements (4) vorgesehen ist, wobei das in der Tasche (10) aufgenommene Anbindungselement (6) und/oder Dämpfungselement (4) in der Tasche (10) relativ zu dieser beweglich angeordnet ist, wobei die Tasche (10) bevorzug ein Material mit einem niedrigen Reibungskoeffizienten aufweist, wobei die Tasche (10) bevorzugt mit der Stützanordnung (2) oder der Halteanordnung (3) verbunden ist.

7. Fußbewegungsbegrenzungsvorrichtung (1) gemäß Anspruch 6, wobei die Fußbewegungsbegrenzungsvorrichtung weiter eine Umhüllung (11) umfasst, die das Dämpfungselement (4) und bevorzugt das zumindest eine Anbindungselement (6, 6') zumindest teilweise umhüllt, wobei die Umhüllung (11) bevorzugt zumindest teilweise innerhalb der Tasche (10) relativ zur Tasche (10) beweglich angeordnet ist, wobei die Umhüllung (11) bevorzugt ein Positionierung des Dämpfungselements (4) und des zumindest einen Anbindungselements (6, 6') relativ zueinander bereitstellt.

8. Fußbewegungsbegrenzungsvorrichtung (1) gemäß einem der Ansprüche 3 bis 7, wobei das Dämpfungselement (4) eine mit einem Dämpfungsfluid gefüllte Aufnahme (15) aufweist, in der ein Auszugskörper relativ zu dieser beweglich aufgenommen ist, wobei der Auszugskörper mit einer sich in einer Auszugsrichtung aus der Aufnahme (15) erstreckenden Zugelement (16) verbunden ist, wobei bevorzugt die Aufnahme (15) proximal angeordnet ist und sich das Zugelement (16) distal aus der Aufnahme (15) erstreckt, wobei das Zugelement (16) gelenkig mit der Halteanordnung (3) verbunden ist, wobei bevorzugt die Anbindung (9) des mindestens eines Anbindungselements (6) an die Aufnahme (15) an einem distalen Ende der Aufnahme (15) angeordnet ist.

9. Fußbewegungsbegrenzungsvorrichtung (1) gemäß einem der Ansprüche 3 - 8, wobei das Anbindungselement (6) und/oder das Dämpfungselement (4) an einem an einer festen Stelle der Halteanordnung (3) angeordneten Drehgelenk und/oder Kugelgelenk gelenkig gehalten ist und eine gelenkige Halterung (5) ausbildet, und/oder die gelenkige Halterung (5) derart ausgebildet ist, dass das Anbindungselement (6) und/oder das Dämpfungselement (4) in einem vorgegebenen Bereich verschiebbar zur Halteanordnung (3) an diese angebunden ist, wobei bevorzugt die Halteanordnung (3) ein Band aufweist, dass an zwei voneinander beabstandeten Stellen an der Halteanordnung (3) befestigt ist, wobei das Anbindungselement (6) und/oder das Dämpfungselement (4), bevorzugt über eine Öse oder Schlaufe, durch welche das Band verläuft, an dem Band gehalten ist.

10. Fußbewegungsbegrenzungsvorrichtung (1) gemäß einem der vorstehenden Ansprüche, wobei oberhalb des Außenknöchels (110) ein Volumenkörper, bevorzugt eine Pelotte (17), zum Abstützen von proximal auf den Außenknöchel (110) angeordnet ist und/oder oberhalb des Innenknöchels (110) ein Volumenkörper, bevorzugt eine Pelotte (17), zum Abstützen von proximal auf den Innenknöchel (110) angeordnet ist.

11. Schuh (50) zum Begrenzen einer Fußbewegung über das Sprunggelenk, umfassend einen Sohlenbereich (52) und einen an den Sohlenbereich (52) angebundenes Schuhoberteil (51),
**gekennzeichnet durch**
eine Fußbewegungsbegrenzungsvorrichtung (1) gemäß einem der vorstehenden Ansprüche.

12. Schuh (50) gemäß dem vorstehenden Anspruch, wobei die Halteanordnung (3) in den Sohlenbereich (52) integriert ist und die Stützanordnung (2) in einen Schaftbereich (53) des Schuhoberteils (51) integriert ist, wobei das Anbindungselement (6) relativ zum Schuhoberteil (51) beweglich ist.

13. Schuh (50) gemäß einem der beiden vorstehenden Ansprüche, wobei die Fußbewegungsbegrenzungsvorrichtung (1) im Wesentlichen komplett in den Schuh (50) integriert ist.

14. Schuh (50) gemäß einem der Ansprüche 11 bis 13, ferner umfassend einen flexiblen Verformungsabschnitt (54) zum Ermöglichen einer Relativbewegung der Stützanordnung (2) relativ zur Halteanordnung (3).

15. Schuh (50) gemäß einem der Ansprüche 11 bis 14, wenn rückbezogen auf eine Fußbewegungsbegrenzungsvorrichtung (1) gemäß Anspruch 5 oder 6, wobei die Tasche (10) fest mit dem Schuhoberteil (51) verbunden ist und bevorzugt in den Schuhoberteil (51) integriert ist, und/oder wobei das Anbindungselement (6) und/oder das Dämpfungselement (4) im Wesentlichen distal des Knöchels (110) angeordnet ist und/oder wobei ein Fenster, bevorzugt ein Sichtfenster, am Schuhoberteil (51) vorgesehen ist, derart ausgebildet, dass von außerhalb des Schuhs (50) eine Sicht auf zumindest einen Teil des Anbindungselements (6) und/oder des Dämpfungselements (4) freigegeben ist.

## Claims

1. A foot movement limiting device (1) for limiting a foot movement via the ankle joint, comprising a support arrangement (2) for supporting on a lower leg or from proximally on the ankle bone (110), a retaining arrangement (3) for retention on a foot (100), preferably on a sole region, and at least one attachment element (6) for limiting a relative movement between the support arrangement (2) and the retaining arrangement (3),
wherein the at least one attachment element (6) is attached to the support arrangement (2) at two different attachment points (7, 7'),
wherein the support arrangement (2) comprises a stretch-resistant region (13) extending posteriorly around from a lateral side, from the first attachment point (7), at least to a medial side, to the second attachment point (7'), or the support arrangement (2) comprises a stretch-resistant region (13) extending anteriorly around from a lateral side, from the first attachment point (7), at least to a medial side, to the second attachment point (7'), wherein the stretch-resistant region (13) is connected at its ends by a stretch-elastic region (14), wherein the stretch-elastic region (14) preferably extends between the first attachment point (7) and the second attachment point (7') counter to the stretch-resistant region (13),
**characterized in that**
the at least one attachment element (6) is, at a point between the two attachment points (7, 7'), held by means of an articulated mounting (5) on the retaining arrangement (3),
wherein the articulated mounting (5) is configured in such a way that the attachment element (6) is attached to the retaining arrangement (3) so as to be displaceable relative to the latter within a predefined range.

2. The foot movement limiting device (1) as claimed in claim 1, wherein the at least one attachment element (6) is attached to the support arrangement (2) at two different attachment points (7, 7'), wherein the at least one attachment element (6) preferably extends from one attachment point (7) to the other attachment point (7'), and the at least one attachment element (6) is, between the two attachment points (7, 7'), held displaceably on the retaining arrangement (3) and/or the attachment points (7, 7') are positioned relative to the attachment element (6) in such a way that a retaining force arising in the attachment element (6) as a result of a foot movement via the ankle joint is divided into a laterally and proximally acting component and a medially and proximally acting component.

3. The foot movement limiting device (1) as claimed in claim 1 or 2, wherein the at least one attachment element (6) is held by way of a damping element (4) on the support arrangement (2) or the retaining arrangement (3), wherein the damping element (4) is preferably fastened to the stretch-resistant region (13) of the support arrangement (2).

4. The foot movement limiting device (1) as claimed in claim 3, wherein the damping element (4) is held displaceably on the at least one attachment element (6), wherein the damping element (4) is attached to the attachment element (6) between the two attachment points (7, 7').

5. The foot movement limiting device (1) as claimed in claim 3 or 4, further comprising a second attachment element (6'), wherein preferably
the first attachment element (6), relative to a central spline (8), preferably a central axis, of the damping element (4), is attached preferably slidably to the damping element (4), preferably at a distance from the central spline (8), preferably from the central axis, on a first side of the damping element (4), and
the second attachment element (6'), relative to the central spline (8), preferably the central axis, of the damping element (4), is attached preferably slidably to the damping element (4), at a distance from the central spline (8), preferably from the central axis, on a second side of the damping element (4),
wherein the distance of the attachments (9, 9') of first attachment element (6) and second attachment element (6') to the damping element (4) is substantially identical relative to the central spline (8), preferably to the central axis,
wherein preferably the first attachment element (6) and the second attachment element (6') are configured together as an individual part, and the individual part is turned back and guided slidably at the first attachment point (7) or the second attachment point (7').

6. The foot movement limiting device (1) as claimed in any of claims 3 to 5, wherein a pocket (10) is provided for at least partially receiving the attachment element (6) and/or the damping element (4), wherein the attachment element (6) and/or damping element (4) received in the pocket (10) is arranged in the pocket (10) in such a way as to be movable relative to the latter, wherein the pocket (10) preferably has a material with a low coefficient of friction, wherein the pocket (10) is preferably connected to the support arrangement (2) or the retaining arrangement (3).

7. The foot movement limiting device (1) as claimed in claim 6, wherein the foot movement limiting device furthermore comprises a sheath (11) which at least partially envelops the damping element (4) and preferably the at least one attachment element (6, 6'), wherein the sheath (11) is preferably at least partially arranged inside the pocket (10) movably relative to the pocket (10), wherein the sheath (11) preferably provides a positioning of the damping element (4) and of the at least one attachment element (6, 6') relative to each other.

8. The foot movement limiting device (1) as claimed in any of claims 3 to 7, wherein the damping element (4) has a receptacle (15) which is filled with a damping fluid and in which a pull-out body is received movably relative to the latter, wherein the pull-out body is connected to a tensioning element (16) extending in a pull-out direction from the receptacle (15), wherein the receptacle (15) is preferably arranged proximally and the tensioning element (16) extends distally from the receptacle (15), wherein the tensioning element (16) is connected in an articulated manner to the retaining arrangement (3), wherein the attachment (9) of the at least one attachment element (6) to the receptacle (15) is preferably arranged at a distal end of the receptacle (15).

9. The foot movement limiting device (1) as claimed in any of claims 3 to 8, wherein the attachment element (6) and/or the damping element (4) is held in an articulated manner on a pivot joint and/or ball joint arranged at a fixed location of the retaining arrangement (3) and forms an articulated mounting (5), and/or the articulated mounting (5) is configured in such a way that the attachment element (6) and/or the damping element (4) is attached to the retaining arrangement (3) so as to be displaceable relative to the latter within a predefined range, wherein the retaining arrangement (3) preferably has a band which is fastened to the retaining arrangement (3) at two locations spaced apart from each other, wherein the attachment element (6) and/or the damping element (4) is held on the band preferably via an eyelet or loop through which the band runs.

10. The foot movement limiting device (1) as claimed in any of the preceding claims, wherein a volume body, preferably a pad (17), is arranged above the lateral malleolus (110), in order to bear from proximally on the lateral malleolus (110), and/or a volume body, preferably a pad (17), is arranged above the medial malleolus (110), in order to bear from proximally on the medial malleolus (110).

11. A shoe (50) for limiting a foot movement via the ankle joint, comprising a sole region (52) and, attached to the sole region (52), a shoe upper (51),
**characterized by**
a foot movement limiting device (1) as claimed in any of the preceding claims.

12. The shoe (50) as claimed in the preceding claim, wherein the retaining arrangement (3) is integrated in the sole region (52) and the support arrangement (2) is integrated in a shaft region (53) of the shoe upper (51), wherein the attachment element (6) is movable relative to the shoe upper (51).

13. The shoe (50) as claimed in either of the preceding two claims, wherein the foot movement limiting device (1) is integrated substantially completely in the shoe (50).

14. The shoe (50) as claimed in any of claims 11 to 13, further comprising a flexible deformation portion (54) in order to permit a relative movement of the support arrangement (2) relative to the retaining arrangement (3).

15. The shoe (50) as claimed in any of claims 11 to 14, wherein the pocket (10) is connected fixedly to the shoe upper (51) and is preferably integrated in the shoe upper (51), and/or wherein the attachment element (6) and/or the damping element (4) is arranged substantially distally from the ankle bone (110), and/or wherein a window, preferably a viewing window, is provided on the shoe upper (51), configured in such a way that a view of at least a part of the attachment element (6) and/or of the damping element (4) is provided from outside the shoe (50).

## Revendications

1. Dispositif de limitation de mouvement de pied (1) pour la limitation d'un mouvement de pied par le biais de la cheville, comprenant un agencement d'appui (2) pour l'appui contre une jambe ou depuis la partie proximale au niveau de l'os de la cheville (110), un agencement de retenue (3) pour la retenue sur un pied (100), de préférence une zone de plante de pied, et au moins un élément de liaison (6) pour la limitation d'un mouvement relatif entre l'agencement d'appui (2) et l'agencement de retenue (3),
dans lequel l'au moins un élément de liaison (6) est lié en deux différents points de liaison (7, 7') à l'agencement d'appui (2), dans lequel l'agencement d'appui (2) comporte une zone (13) rigide en traction, s'étendant depuis un côté latéral du premier point de liaison (7) de part et d'autre postérieurement au moins sur un côté médial au second point de liaison (7'), ou l'agencement d'appui (2) comporte une zone (13) rigide en traction, s'étendant depuis un côté latéral du premier point de liaison (7) de part et d'autre antérieurement au moins sur un côté médial au second point de liaison (7'),
dans lequel la zone (13) rigide en traction est reliée à ses extrémités par une zone (14) élastique en traction, dans lequel la zone (14) élastique en traction s'étend entre le premier point de liaison (7) et le second point de liaison (7') à l'opposé de la zone (13) rigide en traction
**caractérisé en ce que**
l'au moins un élément de liaison (6) est maintenu en un point entre les deux points de liaison (7, 7') par le biais d'un support articulé (5) au niveau de l'agencement de retenue (3),
dans lequel le support (5) articulé est réalisé de telle manière que l'élément de liaison (6) soit lié dans une zone prédéfinie de manière déplaçable par rapport à l'agencement de retenue (3) à celui-ci.

2. Dispositif de limitation de mouvement de pied (1) selon la revendication 1, dans lequel l'au moins un élément de liaison (6) est lié en deux différents points de liaison (7, 7') à l'agencement d'appui (2), dans lequel l'au moins un élément de liaison (6) s'étend de préférence d'un point de liaison (7) à l'autre point de liaison (7') et l'au moins un élément de liaison (6) est maintenu entre les deux points de liaison (7, 7') de manière déplaçable au niveau de l'agencement de retenue (3) et/ou les points de liaison (7, 7') sont positionnés par rapport à l'élément de liaison (6) de telle manière qu'une force de retenue apparaissant à la suite d'un mouvement de pied par le biais de la cheville dans l'élément de liaison (6) soit divisée en une composante agissant en latéral-proximal et une composante agissant en médial-proximal.

3. Dispositif de limitation de mouvement de pied (1) selon la revendication 1 ou 2, dans lequel l'au moins un élément de liaison (6) est maintenu par le biais d'un élément d'amortissement (4) au niveau de l'agencement d'appui (2) ou l'agencement de retenue (3), dans lequel de préférence, l'élément d'amortissement (4) est fixé à la zone (13) rigide en traction de l'agencement d'appui (2).

4. Dispositif de limitation de mouvement de pied (1) selon la revendication 3, dans lequel l'élément d'amortissement (4) est maintenu de manière déplaçable au niveau d'au moins un élément de liaison (6), dans lequel l'élément d'amortissement (4) est lié entre les deux points de liaison (7, 7') à l'élément de liaison (6).

5. Dispositif de limitation de mouvement de pied (1) selon la revendication 3 ou 4, comprenant de plus un second élément de liaison (6'), dans lequel de préférence
le premier élément de liaison (6) est lié par rapport à un spline médial (8), de préférence un axe médian, de l'élément d'amortissement (4) à distance du spline médial (8), de préférence de l'axe médian, sur un premier côté de l'élément d'amortissement (4), de préférence par glissement à l'élément d'amortissement (4), et
le second élément de liaison (6') est lié par rapport au spline médial (8), de préférence l'axe médian, de l'élément d'amortissement (4) à distance du spline médial (8), de préférence l'axe médian, sur un second côté de l'élément d'amortissement (4) de préférence par glissement à l'élément d'amortissement (4),
dans lequel de préférence, la distance des liaisons (9, 9') du premier élément de liaison (6) et du second élément de liaison (6') à l'élément d'amortissement (4) par rapport au spline médian (8), de préférence à l'axe médian est sensiblement de même taille,
dans lequel de préférence, le premier élément de liaison (5) et le second élément de liaison (6') sont réalisés ensemble comme partie individuelle et la partie individuelle est déviée et est guidée par glissement sur le premier point de liaison (7) ou le second point de liaison (7').

6. Dispositif de limitation de mouvement de pied (1) selon l'une quelconque des revendications 3 à 5, dans lequel une poche (10) est prévue pour la réception au moins partielle de l'élément de liaison (6) et/ou de l'élément d'amortissement (4), dans lequel l'élément de liaison (6) reçu dans la poche (10) et/ou l'élément d'amortissement (4) est agencé dans la poche (10) de manière mobile par rapport à celle-ci, dans lequel la poche (10) présente de préférence un matériau avec un coefficient de friction faible, dans lequel la poche (10) est de préférence reliée à l'agencement d'appui (2) ou à l'agencement de retenue (3).

7. Dispositif de limitation de mouvement de pied (1) selon la revendication 6, dans lequel le dispositif de limitation de mouvement de pied comporte en outre une enveloppe (11) qui enveloppe l'élément d'amortissement (4) et de préférence l'au moins un élément de liaison (6, 6') au moins partiellement, dans lequel l'enveloppe (11) est agencée de manière mobile de préférence au moins partiellement dans la poche (10) par rapport à la poche (10), dans lequel l'enveloppe (11) fournit de préférence un positionnement de l'élément d'amortissement (4) et d'au moins un élément de liaison (6, 6') l'un par rapport à l'autre.

8. Dispositif de limitation de mouvement de pied (1) selon l'une quelconque des revendications 3 à 7, dans lequel l'élément d'amortissement (4) présente un logement (15) rempli d'un fluide d'amortissement, dans lequel un corps de rallonge est reçu de manière mobile par rapport à celui-ci, dans lequel le corps de rallonge est relié à un élément de traction (16) s'étendant dans un sens de rallonge depuis le logement (15), dans lequel de préférence, le logement (15) est agencé de manière proximale et l'élément de traction (16) s'étend de manière distale depuis le logement (15), dans lequel l'élément de traction (16) est relié par articulation à l'agencement de retenue (3), dans lequel de préférence la liaison (9) d'au moins un élément de liaison (6) au niveau du logement (15) est agencée à une extrémité distale du logement (15).

9. Dispositif de limitation de mouvement de pied (1) selon l'une quelconque des revendications 3 à 8, dans lequel l'élément de liaison (6) et/ou l'élément d'amortissement (4) est maintenu par articulation sur une articulation rotative et/ou articulation sphérique agencée en un point fixe de l'agencement de retenue (3) et réalise un support (5) articulé, et/ou le support (5) articulé est réalisé de telle manière que l'élément de liaison (6) et/ou l'élément d'amortissement (4) est lié dans une zone prédéfinie de manière déplaçable par rapport à l'agencement de retenue (3) à celui-ci, dans lequel de préférence, l'agencement de retenue (3) présente une bande qui est fixée en deux points espacés l'un de l'autre à l'agencement de retenue (3), dans lequel l'élément de liaison (6) et/ou l'élément d'amortissement (4) est maintenu par la bande de préférence par le biais d'un œillet ou d'une boucle, par laquelle la bande s'étend.

10. Dispositif de limitation de mouvement de pied (1) selon l'une quelconque des revendications précédentes, dans lequel au-dessus de la malléole externe (110), un corps de volume, de préférence une pelote (17), est agencé pour l'appui depuis la partie proximale sur la malléole externe (110) et/ou un corps de volume, de préférence une pelote (17), est agencé au-dessus de la malléole interne (110) pour l'appui depuis la partie proximale sur la malléole interne (110).

11. Chaussure (50) pour la limitation d'un mouvement de pied par le biais de la cheville, comprenant une zone de semelle (52) et une empeigne (51) liée à la zone de semelle (52),
**caractérisée par**
un dispositif de limitation de mouvement de pied (1) selon l'une quelconque des revendications précédentes.

12. Chaussure (50) selon la revendication précédente, dans laquelle l'agencement de retenue (3) est intégré dans la zone de semelle (52) et l'agencement d'appui (2) est intégré dans une zone de tige (53) de l'empeigne (51), dans laquelle l'élément de liaison (6) est mobile par rapport à l'empeigne (51).

13. Chaussure (50) selon l'une quelconque des deux revendications précédentes, dans laquelle le dispositif de limitation de mouvement de pied (1) est intégré sensiblement complètement dans la chaussure (50).

14. Chaussure (50) selon l'une quelconque des revendications 11 à 13, comprenant de plus une section de déformation (54) flexible pour permettre un mouvement relatif de l'agencement d'appui (2) par rapport à l'agencement de retenue (3).

15. Chaussure (50) selon l'une quelconque des revendications 11 à 14, lorsqu'elle se réfère à un dispositif de limitation de mouvement de pied (1) selon la revendication 5 ou 6, dans laquelle la poche (10) est fixement reliée à l'empeigne (51) et est de préférence intégrée dans l'empeigne (51), et/ou dans laquelle l'élément de liaison (6) et/ou l'élément d'amortissement (4) est agencé sensiblement distalement de l'os (110) et/ou dans laquelle une fenêtre, de préférence une fenêtre de visualisation, est prévue sur l'empeigne (51) réalisée de telle manière que depuis l'extérieur de la chaussure (50) une vue sur au moins une partie de l'élément de liaison (6) et/ou de l'élément d'amortissement (4) soit libérée.
